# FASCICULE DE BREVET EUROPEEN

(11) **EP 0 863 760 B1**
(45) Date de publication et mention de la délivrance du brevet: **05.12.2001**
(21) Numéro de dépôt: 96931876.5
(22) Date de dépôt: 19.09.1996
(51) Int. Cl.: A61K 31/58, A61K 31/585

(54) **UTILISATION DES COMPOSES ANTIMINERALOCORTICOIDES CONTRE LE SYNDROME DE SEVRAGE DES NARCOTIQUES**
VERWENDUNG VON ANTIMINERALCORTICOIDVERBINDUNGEN GEGEN DAS DROGENENTZUGSSYNDROM
USE OF ANTIMINERALOCORTICOID COMPOUNDS AGAINST DRUG WITHDRAWAL SYNDROME

(30) Priorité: 21.09.1995 FR 9511086
(43) Date de publication de la demande: 16.09.1998
(73) Titulaire: Aventis Pharma S.A., 92160 Antony (FR)
(72) Inventeur: PETIT, Francis, F-92700 Colombes (FR); PHILIBERT, Daniel, F-94210 La-Varenne-Saint-Hilaire (FR); GOEDERS, Nick, Shreveport, LA 71104-4414 (US)
(86) Numéro de dépôt international: FR9601459
(87) Numéro de publication internationale: WO9710827

(56) Documents cités:
- FARMAKOL. TOKSIKOL., vol. 41, no. 5, 1978, pages 541-544, XP000574604 Z.I.ELTSOVA ET AL.: "The action of morphine and amidopyrine against the background of the mineralocorticoid hormone or its antagonist."
- EUR. J. PHARMACOL., vol. 56, no. 3, 1979, pages 197-205, XP000574608 C.L. WONG ET AL: "the effect of stress and adrenalectomy on morphine analgesia and naloxone potency in mice."
- J. PHARMACOL. EXP. THER., vol. 236, no. 1, 1986, pages 157-165, XP000574609 B.S. NEAL ET AL.: "Mianserin attenuates naloxone-precipitated withdrawal signs in rats acutely or chronically dependent upon morphine."
- FED. PROC., vol. 40, no. 5, 1981, pages 1502-1507, XP002007591 R.J. VALENTINO ET AL.: "Physiological and behavioural approaches to the study of the quasi-morphine withdrawal syndrome."
- EUR.J. PHARMACOL., vol. 263, no. 1-2, 1994, pages 149-156, XP000575461 E. RONKEN ET AL.: "Glucocorticoid and mineralocorticoid receptors differentially modulate cultured dopaminergic neurons of rat ventral mesencephalon."

## Description

Application des composés ayant une activité antiminéralocorticoïde pour la préparation de médicaments destinés à la prévention ou au traitement des manifestations liées à la dépendance ou au syndrome de sevrage spontané ou précipité, provoqué par les narcotiques et les compositions les renfermant.

Les produits possédant une activité antiminéralocorticoïde sont connus comme pouvant être utilisés comme médicaments. Ils sont, en particulier, antagonistes de l'aldostérone et ils augmentent la diurèse hydrosodée avec conservation du potassium organique ; ils présentent, en outre, pour certains, l'avantage d'être dénués d'effets hormonaux secondaires, en particulier d'effets anti-androgène et anti-estrogène. Ils peuvent donc être utilisés pour lutter, notamment, contre l'hypertension artérielle et les insuffisances cardiaques.

Il y a deux types majeurs de récepteurs du glucocorticoïde au niveau du système nerveux central, le récepteur de type I et le récepteur de type II (R. Anima et col. J. Comp. Neurol. 313 (1991) 522-528 ; Neuroscience 39 (1990) 579-604).

Le récepteur de type I, au niveau du cerveau, est identique au récepteur minéralocorticoïde classique trouvé au niveau du rein, et il présente une haute affinité et une faible capacité de liaison pour les glucocorticoïdes endogènes. En d'autres termes, un antiminéralocorticoïde se comporte au niveau du système nerveux central comme un antiglucocorticoïde de type I.

La demanderesse a mis en évidence l'application nouvelle et inattendue de ces produits, énoncée plus haut.

Il a été précédemment montré que les glucocorticoïdes (type dexaméthasone) antagonisent l'activité analgésique de la morphine alors qu'un antagoniste des glucocorticoïdes, type 17β-hydroxy 11β-(4-diméthylaminophényl) 17α-(prop-1-ynyl) estra 4,9-dièn-3-one, ou une surrénalectomie, potentialise cette activité (Capasso et al. Life Science 51 139 (1992), Ratka et al. Neuroendocrinology 49 439 (1988) Pieretti et al. Gen. Pharmacol. 22 929 (1991)).

Cependant, à la connaissance de la demanderesse, personne n'a mis en évidence l'activité d'un antiminéralocorticoïde vis-à-vis des effets indésirables des opiacés et en particulier de l'induction d'un état de dépendance physique ou psychique et du syndrome de sevrage qui est associé à cet état. Ces phénomènes de dépendance et de sevrage impliquent des mécanismes centraux complexes, multiples et différents de ceux qui sont observés dans l'activité analgésique des opiacés.

D'autre part, des données récentes ont été rapportées sur le rôle important que pouvaient jouer les glucocorticoïdes endogènes dans les manifestations du sevrage narcotique, de même que dans les phénomènes de dépendance induits par les opiacés ou la cocaïne. Ainsi, un hypercortisolisme a été observé chez l'homme au cours d'essais cliniques, lors d'un sevrage spontané ou précipité par la naloxone consécutif à la prise d'héroïne ou de morphine (Cami et al. Br. J. Addict 87 1145 (1992), Higgins et al. Drug Alcohol Depend. 30 13 (1992). D'autres éléments rapportés chez l'animal montrent, une activation de l'axe hypothalamo-surrénalien par la cocaïne (Borowsky and Kuhn, J. Pharmacol. Exp. Ther. (1991) 256, 204) administrée en traitement aigu ou répété avec une augmentation des taux plasmatiques de corticostérone et d'ACTH (Moldow and Fischman, Peptides 8 819 (1987), Yang et al. Pharmacol. Biochem Behau. 41 643 (1992), Saphier et al. Neuroendocrinology 57 54 (1993)) consécutive à une médiation d'origine monoaminergique (dopamine par exemple). Par exemple l'implication du système dopaminergique semble confirmée par le fait que l'halopéridol et le métoclopramide (antagonistes dopaminergiques) s'opposent respectivement à l'élévation des taux de corticostérone induite par la cocaïne et au phénomène de sevrage morphinique (Ramaswamy and Bapna, Life Science 40 807 (1987)).

Ces données semblent montrer que les glucocorticoïdes endogènes pourraient intervenir dans les phénomènes de sevrage et de dépendance, au même titre que des mécanismes dopaminergiques mais à une étape plus en amont que ces derniers.

Ces différents éléments ont justifié l'étude d'antagonistes des minéralocorticoïdes en particulier vis-à-vis des phénomènes de dépendance psychique et physique ou du syndrome de sevrage morphinique précipité par la naioxone chez l'animal puisqu'aucune donnée n'est actuellement disponible sur l'activité de cette classe thérapeutique dans cet axe.

En effet, si une élévation des taux de glucocorticoïdes endogènes a été rapportée dans les phénomènes de sevrage opiacé, il n'a pas été démontré que cette augmentation pouvait avoir un retentissement physiopathologique et qu'en particulier le blocage de ces glucocorticoïdes endogènes au niveau de leurs récepteurs par un antiminéralocorticoïde pouvait se traduire par un effet bénéfique sur les phénomènes de dépendance psychique et physique et sur les manifestations du syndrome de sevrage.

C'est ainsi que la demanderesse a mis en évidence une application nouvelle et inattendue des antiminéralocorticoïdes.

La présente invention a donc pour objet l'application des composés ayant une activité antiminéralocorticoïde pour la préparation de médicaments destinés à la prévention ou au traitement des manifestations liées à la dépendance ou au syndrome de sevrage spontané ou précipité provoqué par les narcotiques ou mélanges de narcotiques.

On entend par composés ayant une activité antiminéralocorticoïde,
soit les composés qui sont des antagonistes du récepteur aldostérone, lesquels composés sont des inhibiteurs compétitifs de la liaison du stéroïde à son récepteur, empêchant donc l'hormone naturelle d'exercer son activité,
soit les composés qui inhibent la biosynthèse de l'aldostérone, en inhibant en particulier la 18-hydroxylase. En effet, l'étape d'oxydation en 18 constitue la dernière étape de la biosynthèse de l'aldostérone et une inhibition sélective de cette étape permet, en principe, d'éviter d'inhiber la biosynthèse d'autres hormones stéroïdes essentielles telles que le cortisol ou l'androstanedione. Ces composés sont essentiellement représentés par les composés de la figure (Iⱼ) décrits plus bas dans laquelle R₄ⱼ est un groupement alkényle ou alkynyle et R₅ⱼ est soit un radical hydroxyle, soit un atome d'hydrogène.

On entend par narcotiques, toutes drogues entrainant un phénomène de dépendance physique et psychique et dont l'arrêt spontané ou précipité entraîne un syndrome de sevrage. On peut citer :
**1)** les morphinomimétiques naturels tels que :
   a) les alcaloïdes de l'opium, par exemple la morphine,
   b) les alcaloïdes dérivés de la morphine, par exemple l'héroïne ou la codéine,
**2)** les morphinomimétiques de synthèse tels que :
   a) les dérivés de la pipéridine, par exemple la péthidine ou
   b) la méthadone et ses dérivés, par exemple le dextromoramide,
**3)** la cocaïne,
ainsi que toutes les associations renfermant deux ou plusieurs de ces produits narcotiques.

La présente invention a plus spécialement pour objet l'application des composés ayant une activité antiminéralocorticoïde pour la préparation de médicaments destinés à la prévention ou au traitement des manifestations liées à la dépendance ou au syndrome de sevrage spontané ou précipité provoqué par les narcotiques morphinomimétiques choisis parmi l'héroïne, la morphine et la méthadone.

La présente invention a plus spécialement pour objet l'application des composés ayant une activité antiminéralocorticoïde pour la préparation de médicaments destinés à la prévention ou au traitement des manifestations liées à la dépendance ou au syndrome de sevrage spontané ou précipité provoqué par la cocaïne.

La présente invention a plus particulièrement pour objet l'application telle que définie précédemment, caractérisée en ce que les composés ayant une activité antiminéralocorticoïde répondent à la formule générale (I) : 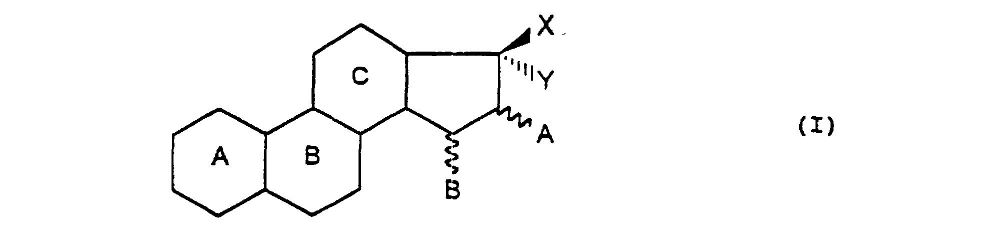 dans laquelle les cycles A, B et C ont l'une des structures suivantes : 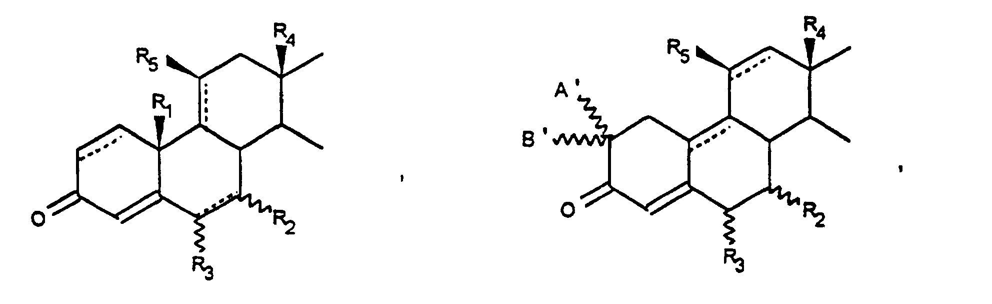 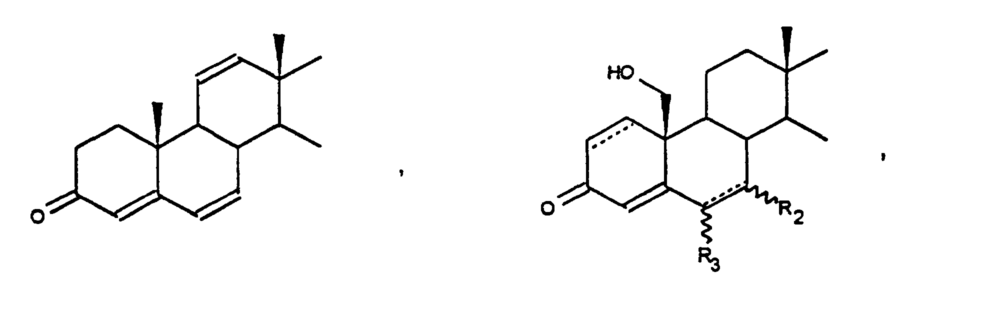 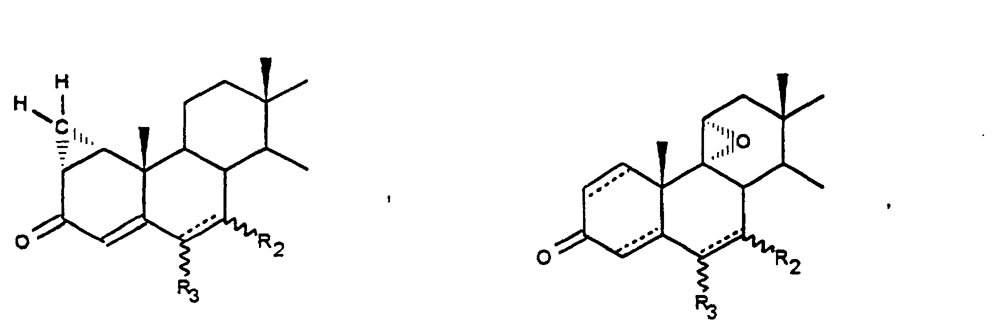 et dans laquelle :
soit X et Y représentent les groupements : 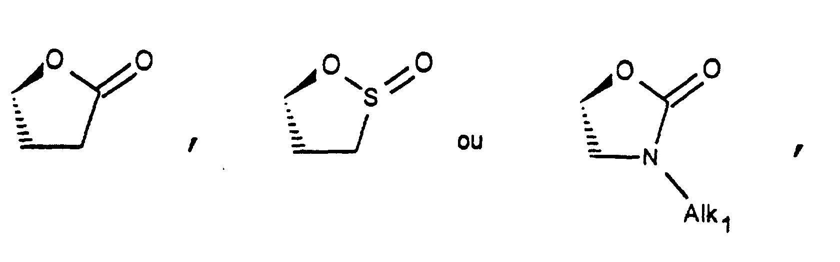 Alk₁ représentant un groupement alkyle renfermant au plus 8 atomes de carbone,
soit X représente un radical hydroxyle, acétyloxy, propionyloxy, méthoxy ou éthoxy et Y représente un radical CH₂CH₂CO₂M, CH₂CH₂SO₂M ou CH₂CH₂CH₂OH, M étant un atome d'hydrogène, un atome de métal alcalin ou un radical ammonium,
soit X représente un radical COCH₂Z, dans lequel Z représente un atome d'hydrogène, un radical hydroxyle ou un radical acyloxy renfermant de 1 à 18 atomes de carbone, et Y représente un atome d'hydrogène,
soit X représente un radical 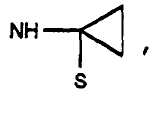 S étant un radical alkyle renfermant au plus 8 atomes de carbone, ou un atome d'hydrogène et Y représente un atome d'hydrogène, A et B sont des atomes d'hydrogène ou forment ensemble un pont méthylénique en position 15α, 16α ou 15β, 16β, A' et B' sont des atomes d'hydrogène, des radicaux alkyle renfermant de 1 à 4 atomes de carbone, ou forment avec le carbone qui les portent un radical cyclopropyle, R₁ représente un radical méthyle ou un groupement 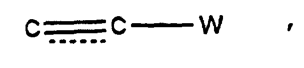 dans lequel W représente
soit un atome d'hydrogène,
soit un radical alkyle renfermant au plus 8 atomes de carbone éventuellement substitué par un radical hydroxyle, carboxy libre, estérifié ou salifié, amino, tritylamino, chloroacétylamino, trifluoroacétylamino, halogène, monoalkylamino, dialkylamino, chaque radical alkyle renfermant au plus 8 atomes de carbone,
soit un radical aryle ou aralkyle renfermant au plus 14 atomes de carbone, éventuellement substitué par un radical hydroxyle, carboxy libre estérifié ou salifié, amino, monoalkylamino, dialkylamino, alkyle, alkoxy ou alkylthio, chaque radical alkyle renfermant au plus 8 atomes'de carbone,
soit un atome d'halogène,
soit un radical trialkylsilyle, chaque radical alkyle renfermant au plus 8 atomes de carbone,
   R₂ et R₃ sont tels que
soit R₂ et R₃ forment ensemble un pont méthylénique en position 6α,7α ou 6β,7β,
soit R₂ et R₃ sont des atomes d'hydrogène,
soit R₃ est un atome d'hydrogène et R₂ représente un groupement SCOCH₃, CO₂Alk, Alk étant un radical alkyle renfermant au plus 8 atomes de carbone, alkyle, alkényle ou alkynyle renfermant renfermant au plus 8 atomes de carbone et éventuellement substitués par un radical hydroxyle, carboxy libre, estérifié ou salifié, halogène, amino, monoalkylamino, dialkylamino, chaque radical alkyle renfermant au plus 8 atomes de carbone, R₄ représente un radical alkyle, alkényle ou alkynyle renfermant au plus 8 atomes de carbone,
   R₅ représente soit un radical allényle, soit un radical hydroxyle, soit un atome d'hydrogène, les traits en pointillés représentent une seconde liaison éventuelle, les traits ondulés indiquent que les substituants sont en position α ou β, ainsi que les sels des produits de formule (I) avec les acides et les bases pharmaceutiquement acceptables.

Par groupement alkyle renfermant au plus 8 atomes de carbone, on entend les radicaux alkyles linéaires ou ramifiés tels que méthyle, éthyle, n-propyle, iso-propyle, butyle, isobutyle, sec-butyle, tert-butyle, pentyle, isopentyle, sec-pentyle, tert-pentyle, néopentyle, hexyle, isohexyle, sec-hexyle, tert-hexyle, heptyle ou octyle.

On préfère les radicaux alkyle ayant au plus 4 atomes de carbone et notamment les radicaux méthyle, éthyle, propyle et isopropyle.

Lorsque R₄ est un groupement alkyle, il s'agit tout particulièrement du radical méthyle.

L'atome de métal alcalin que peut représenter M est de préférence le sodium, le potassium ou le lithium.

Par radical acyloxy, on entend notamment les radicaux formyloxy, acétoxy, propionyloxy, butyryloxy ou benzoyloxy.

Comme valeurs préférées de W, on peut citer l'atome d'hydrogène et les radicaux alkyles renfermant de 1 à 8 atomes de carbone éventuellement substitués par les radicaux tels que décrits précédemment, et tout particulièrement le radical méthyle.

Comme valeur préférée de R₁, on peut citer le groupement C≡C-H et C≡C-Me.

Le terme carboxy éventuellement estérifié désigne les radicaux alkyloxycarbonyle renfermant au plus 9 atomes de carbone, tels que par exemple les radicaux méthoxycarbonyle, éthoxycarbonyle, propoxycarbonyle, n-butyloxycarbonyle, tert-butyloxycarbonyle ou encore benzyloxycarbonyle.

Les termes monoalkylamino et dialkylamino désignent le radical amino substitué par un ou deux radicaux alkyle tels que définis ci-dessus. Il s'agit notamment des radicaux méthylamino et diméthylamino.

Par atome d'halogène, on entend tout particulièrement les atomes de chlore et de brome.

Les radicaux aryle et aralkyle que peuvent représenter W sont de préférence un radical phényle, benzyle ou phénétyle.

Le terme alkoxy désigne des radicaux alkoxy renfermant de 1 à 8 atomes de carbone tel que par exemple méthoxy ou éthoxy.

Le terme alkylthio désigne des radicaux alkylthio renfermant de 1 à 8 atomes de carbone tels que par exemple méthylthio ou éthylthio.

Le terme alkényle désigne un radical alkényle linéaire ou ramifié tel que par exemple les radicaux vinyle, allyle, 1-propényle, butényle, pentényle ou hexenyle.

Parmi les radicaux alkényle, on préfère ceux à 4 atomes de carbone tels que les radicaux allyle, propényle ou butényle.

Le terme alkynyle désigne un radical alkynyle linéaire ou ramifié, ayant au plus 12 atomes de carbone tel que les radicaux éthynyle, propargyle, butynyle, pentynyle ou hexynyle.

Parmi les radicaux alkynyle, on préfère ceux à 4 atomes de carbone tels que le radical propargyle.

Lorsque R₄ est un groupement alkényle ou alkynyle, il s'agit tout particulièrement des radicaux CH₂-C≡CH ou CH₂-CH=CH₂.

Lorsque R₃ est un atome d'hydrogène et lorsque le trait en pointillé ne représente pas une double liaison, R₂ se trouve de préférence en position α.

Lorsque les produits de formule (I) comportent une fonction carboxy, celle-ci peut être salifiée. Parmi les sels possibles, on peut citer par exemple les sels de sodium, de potassium, de lithium, de calcium, de magnésium ou d'ammonium. on peut citer, par les bases organiques, la méthylamine, la propylamine, la triméthylamine, la diéthylamine, la triéthylamine, la N,N-diméthyléthanolamine, le tri-(hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.

Lorsque les produits de formule (I) comportent une fonction salifiable par un acide et notamment une fonction amino, on obtient des sels d'addition avec les acides.

L'invention s'étend naturellement aux sels d'addition avec les acides des composés de formule (I), salifiables, comme par exemple les sels formés avec les acides chlorhydrique, bromhydride, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques.

Les produits de formule (I) sont connus ou sont préparés selon les méthodes connues de l'homme du métier.

La présente invention a plus précisément pour objet l'application telle que définie précédemment, caractérisée en ce que les composés ayant une activité antiminéralocorticoïde répondent à la formule générale (Iₐ) : 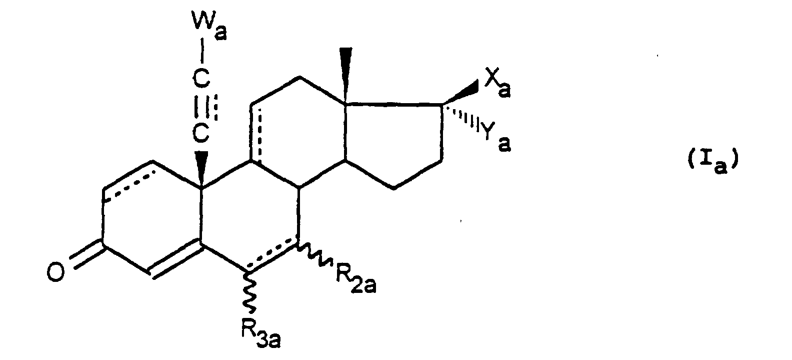 dans laquelle :
soit Xₐ et Yₐ représentent les groupements 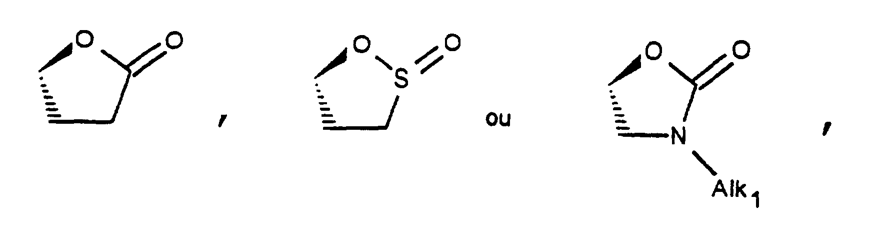 Alk₁ étant tel que défini précédemment,
soit Xₐ représente un radical hydroxyle, acétyloxy, propionyloxy, méthoxy ou éthoxy et Yₐ représente un radical CH₂CH₂CO₂M, CH₂CH₂SO₂M ou CH₂CH₂CH₂OH, M étant tel que défini précédemment,
   R₂ₐ, R₃ₐ et Wₐ ont respectivement les même valeurs que R₂, R₃ et W telles que définies précédemment et les traits pointillés ou ondulés gardent la même signification que précédemment.

La présente invention a tout particulièrement pour objet l'application telle que définie précédemment des produits répondant à la formule générale (Iₐ) choisis dans la liste suivante :
- γ-lactone de l'acide 10β-éthynyle 17β-hydroxy 3-oxo-19-nor-17α-pregna-4,9(11)-diène-21-carboxylique,
- γ-lactone de l'acide 17β-hydroxy 3-oxo-10β-(1-propynyle)-19-nor-17α-pregna-4,9(11)-diène-21-carboxylique,
- γ-lactone de l'acide 17β-hydroxy-3-oxo-10β-(1-propynyl)-19-nor-17α-pregn-4-ène-21-carboxylique,
- γ-lactone de l'acide 10β-éthynyle,17β-hydroxy 3-oxo-19-nor-17α-pregn-4-ène-21-carboxylique.

Les produits de formule générale (Iₐ) sont décrits et préparés dans les demandes de brevets EP 0176399-A1 et EP 0237397-A1 et par les méthodes connues par l'homme du métier.

La présente invention a plus précisément pour objet l'application telle que définie précédemment, caractérisée en ce que les composés ayant une activité antiminéralocorticoïde répondent à la formule générale (I_{b}) : 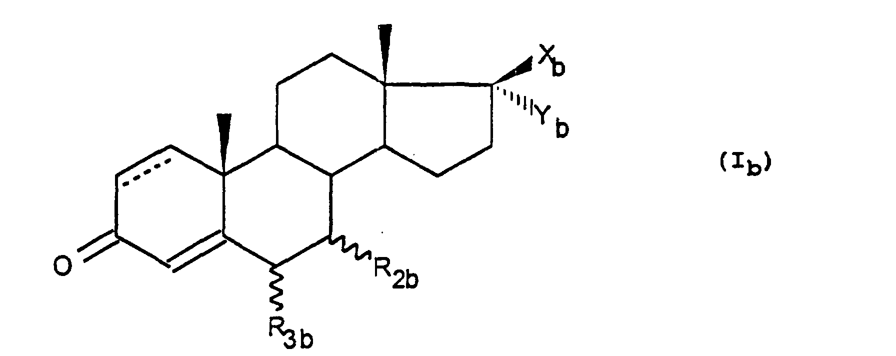 dans laquelle :
soit X_{b} et Y_{b} représentent les groupements 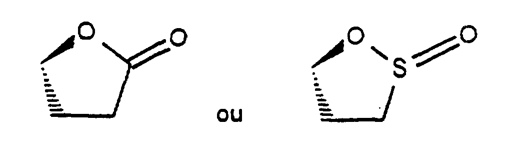
soit X_{b} représente un radical hydroxyle et Y_{b} représente un radical CH₂CH₂CO₂M, M étant tel que défini précédemment,
   R_{2b} et R_{3b} sont tels que
ou bien R_{2b} représente un radical alkyle, alkényle ou alkynyle éventuellement substitués tels que définis précédemment et R_{3b} est un atome d'hydrogène,
ou bien R_{2b} et R_{3b} forment ensemble un pont méthylénique en position 6α,7α ou en position 6β,7β.

Lorsque R_{2b} est un groupement alkyle, il s'agira tout particulièrement du radical propyle en position α.

La présente invention a tout particulièrement pour objet l'application telle que définie précédemment, des produits répondant à la formule générale (I_{b}) choisis dans la liste suivante :
- (17R)-6β,7β-méthylène-2'-oxydospiro-(androst-4-ène-17,5'-(1',2')-oxathiolane)-3-one
- (17R)-6α,7α-méthylène-2'-oxydospiro-(androst-4-ène-17,5'-(1',2')-oxathiolane)-3-one
- (17R)-7α-méthyl-2'-oxydospiro-(androst-4-ène-17,5'-(1',2')-oxathiolane)-3-one
- (17R)-7α-n-propyl-2'-oxydospiro-(androst-4-ène-17,5'-(1',2')-oxathiolane)-3-one
- γ-lactone de l'acide 17β-hydroxy-3-oxo-7α-propyl-17α-pregna-1,4-diène-21-carboxylique,
- 17β-hydroxy-3-oxo-7α-propyl-17α-pregna-1,4-diène-21-carboxylate de potassium,
- γ-lactone de l'acide 17β-hydroxy-3-oxo-7α-propyl-(17α)-pregn-4-ène-21-carboxylique,
- 17β-hydroxy-3-oxo-7α-propyl-(17α)-pregn-4-ène-21-carboxylate de potassium.

Les produits de formule (I_{b}) sont préparés dans les demandes de brevets EP 0018245A, EP 0055170A, FR 2344286, FR 2421913, FR 2465749, et par les méthodes connues par l'homme du métier.

La présente invention a plus précisément pour objet l'application telle que définie précédemment, caractérisée en ce que les composés ayant une activité antiminéralocorticoïde, répondant à la formule générale (I_{c}) : 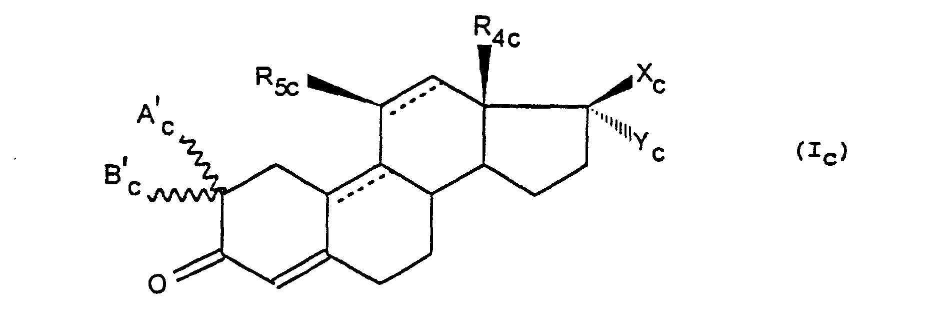 dans laquelle
soit X_{c} et Y_{c} représentent un groupement 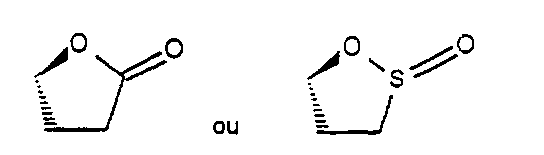
soit X_{c} représente un radical hydroxyle et Y_{c} représente un groupement CH₂CH₂CO₂M ou CH₂CH₂SO₂M, M étant tel que défini précédemment,
soit X_{c} représente un radical COCH₂Z dans lequel Z est tel que défini précédemment et Y_{c} est un atome d'hydrogène,
   A'_{c} et B'_{c} ont respectivement les mêmes valeurs que A' et B' telles que définies précédemment,
   R_{4c} est un radical méthyle ou éthyle, R_{5c} est soit un atome d'hydrogène, soit un radical allényle, étant entendu que lorsque R_{5c} est un radical allényle, A'_{c} et B'_{c} sont des atomes d'hydrogène, R_{4c} est un radical méthyle, X_{c} et Y_{c} forment ensemble un groupement 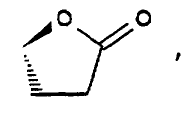 les traits en pointillés en position 9-10 forment une seconde liaison, et ceux en position 11-12 ne forment pas une seconde liaison.

La présente invention a tout particulièrement pour objet l'application telle que définie précédemment des produits répondant à la formule générale (I_{c}) choisis dans la liste suivante :
- 2,2-diméthyl 19-nor pregn-4-ène-3,20-dione,
- 21-acétoxy 2,2-diméthyl 19-nor-pregn-4-ène 3,20-dione,
- 2,2-diméthyl 21-hydroxy 19-nor-pregn-4-ène 3,20-dione,
- 2,2-diméthyl 19-nor-pregna-4,9-diène 3,20-dione,
- 21-acétoxy 2,2-diméthyl 19-nor-pregna-4,9-diène 3,20-dione,
- 2,2-diméthyl 21-hydroxy 19-nor-pregna-4,9-diène 3,20-dione,
- 2,2-diméthyl 19-nor-pregna-4,9,11-triène 3,20-dione,
- 21-acétoxy 2,2-diméthyl 19-nor-pregna-4,9,11-triène 3,20-dione,
- 2,2-diméthyl 21-hydroxy 19-nor-pregna-4,9,11-triène 3,20-dione,
- (17R) 2'-oxydospiro-(estra-4,9-diène-17,5'-(1',2')-oxathiolane) 3-one,
- (17R) 2'-oxydospiro-(estra-4,9,11-triène-17,5'-(1',2')-oxathiolane) 3-one,
- (17R) 11β-hydroxy 2'-oxydospiro-(estra-4,9-diène-17,5'-(1',2')-oxathiolane) 3-one,
- 2,2-diméthyl-13-éthyl-21-hydroxy-18,19-dinor-pregn-4-ène 3,20-dione,
- γ-lactone de l'acide 11β-allényl-17β-hydroxy-3-oxo-19-nor-17α-pregna-4,9-diène-21-carboxylique.

Les produits de formule générale (I_{c}) sont décrits et préparés dans les demandes de brevets FR 2364655, FR 2374037, EP 0012641, dans la publication suivante : G. AUZOU et al. J. Med. Chem. (1993) 36 2404-2407, et par les méthodes connues par l'homme du métier.

La présente invention a plus précisément pour objet l'application telle que définie précédemment caractérisé en ce que les composés ayant une activité antiminéralocorticoïde répondent à la formule générale (I_{d}) : 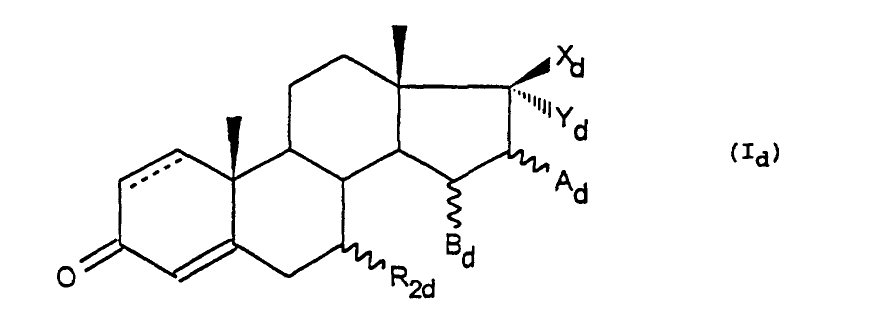 dans laquelle :
soit X_{d} et Y_{d} représentent le groupement 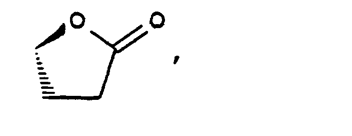
soit X_{d} représente un radical hydroxyle et Y_{d} représente un radical CH₂CH₂CO₂M, M étant tel que défini précédemment,
   A_{d} et B_{d} ont respectivement les mêmes valeurs que A et B telles que définies précédemment,
   R_{2d} représente soit un radical thioacétyle, soit un radical CO₂Alk, Alk étant un radical alkyle renfermant au plus 8 atomes de carbone. Il s'agit de préférence de méthyle, éthyle et isopropyle, et les traits pointillés ou ondulés gardent la même signification que précédemment.

La présente invention a tout particulièrement pour objet l'application telle que définie précédemment des produits répondant à la formule générale (I_{d}) choisis dans la liste suivante :
- γ-lactone de l'acide 7α-acétylthio-17β-hydroxy-15β,16β-méthylène-3-oxo-17α-pregna-1,4-diène-21-carboxylique (Mespirénone),
- γ-lactone de l'acide 17β-hydroxy-7α-méthoxycarbonyle-15β,16β-méthylène-3-oxo-17α-pregn-4-ène-21-carboxylique (ZK91587),
- γ-lactone de l'acide 7α-acétylthio-17β-hydroxy-3-oxo-17α-pregn-4-ène-21-carboxylique (Spironolactone),
- γ-lactone de l'acide 17β-hydroxy-7α-methoxycarbonyle-3-oxo-17α-pregn-4-ène-21-carboxylique (Mexrénone),
- sel de potassium de l'acide 17β-hydroxy-7α-méthoxycarbonyle-3-oxo-pregn-4-ène-21-carboxylique.

Les produits de formule (I_{d}) sont commerciaux ou sont préparés ou décrits dans les demandes de brevets ou publications suivantes :
- Mespirénone : Drug of the Future Vol. 12 n° 1 (1987) 27,
- Mexrénone : G.B. Cutler et al. J. Pharmacol. and Exp. Ther. (1979) 209 144,
- ZK 91587 : H.J. Grill et al. J. Ster. Biochem, 23 (Suppl.) Abst. 19 (1985),
- Spironolactone : J.A. Cella et C.M. Kawaga J. Am. Chem. Soc. (1957) 79 4808,
- Mexrenoate de K. : L.M. Hoffmann et al. The Journal of Pharmacol. and Exp. Ther. (1977) 102 (3) 762,
   ou selon les méthodes connues par l'homme du métier.

La présente invention a plus précisément pour objet l'application telle que définie précédemment, caractérisée en ce que les composés ayant une activité antiminéralocorticoïde répondent à la formule générale (Iₑ) : 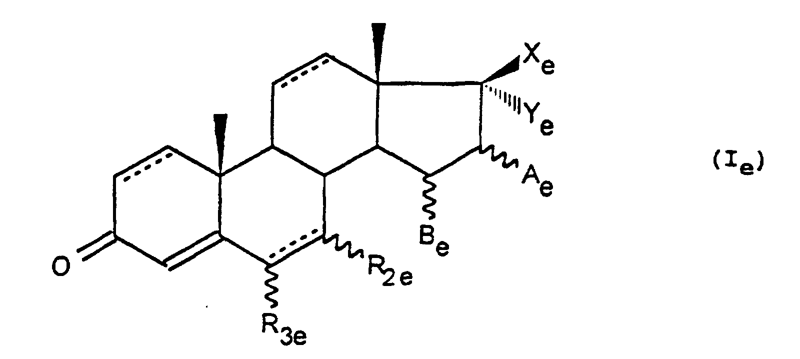 dans laquelle :
soit Xₑ et Yₑ représentent le groupement 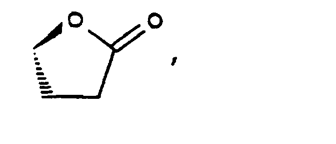
soit Xₑ représente un radical hydroxyle et Ye représente un radical CH₂CH₂CO₂M, M étant tel que défini précédemment,
   R₂ₑ ou R₃ₑ sont tels que :
   ou bien R₂ₑ et R₃ₑ forment ensemble un pont méthylénique en position 6α, 7α ou 6β,7β,
   ou bien R₂ₑ et R₃ₑ sont des atomes d'hydrogène,
      Aₑ et Bₑ ont respectivement les mêmes valeurs que A et B telles que définies précédemment, les traits pointillés ou ondulés gardant leur signification précédente.

La présente invention a tout particulièrement pour objet l'application telle que définie précédemment des produits répondant à la formule générale (Iₑ), choisis dans la liste suivante :
- γ-lactone de l'acide 17β-hydroxy-6β,7β,15β,16β-diméthylène-3-oxo-17α-pregna-1,4-diène-21-carboxylique (Spirorénone),
- γ-lactone de l'acide 17β-hydroxy-6β,7β,15β,16β-diméthylene-3-oxo-17α-pregna-4-ène-21-carboxylique (dihydrospirorénone),
- γ-lactone de l'acide 17β-hydroxy-3-oxo-17α-pregna-4,6,11-triène-21-carboxylique,
- γ-lactone de l'acide 17β-hydroxy-3-oxo-17α-pregna-4,6-diène-21-carboxylique (Canrénone),
- sel de potassium de l'acide 17β-hydroxy-3-oxo-17α-pregna-4,6-diène-21-carboxylique (Canrénoate de potassium),
- γ-lactone de l'acide 17β-hydroxy-6β,7β-méthylène-3-oxo-17α-pregn-4-ène-21-carboxylique (Prorénone),
- sel de potassium de l'acide 17β-hydroxy-6β,7β-méthylène-3-oxo-17α-pregn-4-èn-21-carboxylique (proténoate de potassium).

Les produits de formule (Iₑ) sont commerciaux ou sont préparés ou décrits dans les demandes de brevet ou publications suivantes :
- Spirorénone : W. Losert et al. Arzneim-Forsch/Drug. Res. (1986) 36 1583,
- Dihydrospirorénone : DE 2652761-A,
- Canrénoate de K : L.E. Ramsay et al. Adrenal Steroïd Antagonism. Ed. M.K. Agarwal Berlin, N.Y. 1984,
- Prorénone, Prorénoate de K. : J. Casals-Stenzel et al. Arch. Pharmacol. Suppl. 316 (1981) R49,
- γ-lactone de l'acide 17β-hydroxy-3-oxo-17α-pregna-4,6,11-triène-21-carboxylique, Anu. Drug. Data Rep. (1985) 7 (2) 94.

La présente invention a tout particulièrement pour objet l'application telle que définie précédemment, caractérisée en ce que les composés ayant une activité antiminéralocorticoïde répondent à la formule générale (I_{f}) : 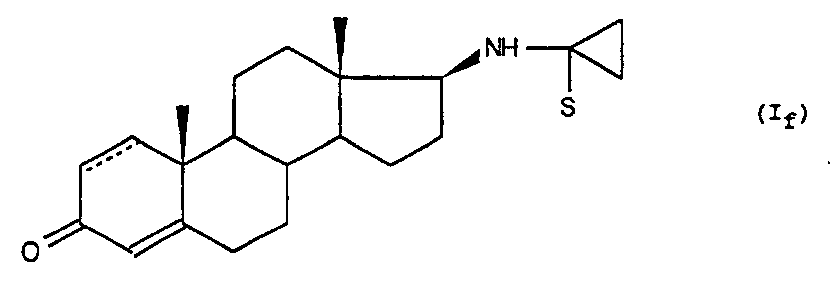 dans laquelle S représente un radical alkyle renfermant de 1 à 8 atomes de carbone, ou un atome d'hydrogène.

Les produits de formule générale (I_{f}) sont décrits dans la demande de brevet EP 402857-A.

La présente invention a plus particulièrement pour objet l'application telle que définie précédemment, caractérisée en ce que les composés ayant une activité antiminéralocorticoïde répondent à la formule générale (I_{g}) : 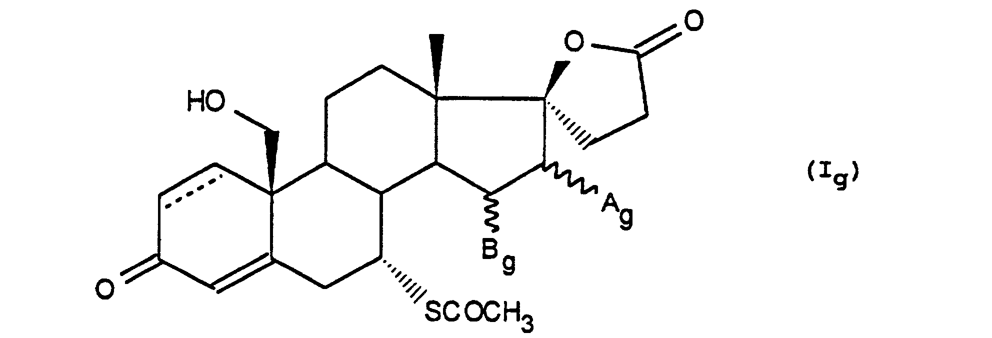 dans laquelle A_{g} et B_{g} ont respectivement les mêmes valeurs que A et B telles que définies précédemment, les traits pointillés ou ondulés gardant la même signification que précédemment.

Les produits de formule générale (I_{g}) sont décrits dans Anu. Drug. Data Rep. (1986) 8 (9) 824.

La présente invention a plus particulièrement pour objet l'application telle que définie précédemment, caractérisée en ce que les composés ayant une activité antiminéralocorticoïde répondant à la formule générale (Iₕ) : 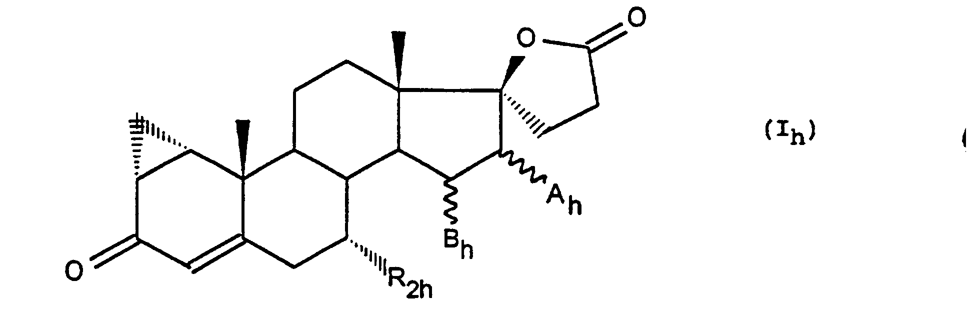 dans laquelle Aₕ, Bₕ et R₂ₕ ont respectivement les mêmes valeurs que A, B et R₂ telles que définies précédemment.

Les produits de formule générale (Iₕ) sont décrits dans Anu. Drug. Data Rep. (1985) 7(5) 295, (1986) 8(2) 152.

La présente invention a plus particulièrement pour objet l'application telle que définie précédemment, caractérisée en ce que les composés ayant une activité antiminéralocorticoïde répondent à la formule générale (Iᵢ) : 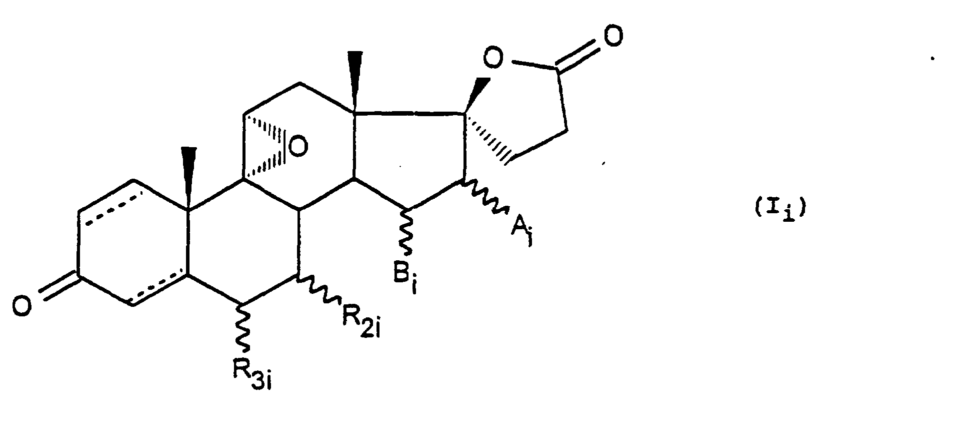 dans laquelle Aᵢ, Bᵢ, R₂ᵢ et R₃ᵢ ont respectivement les mêmes valeurs que A, B, R₂ et R₃ telles que définies précédemment.

Les produits de formule générale (Iᵢ) sont décrits dans Anu. Drug. Data Rep. (1985) 7(5) 295, (1986) 8(9) 824.

La présente invention a plus précisément pour objet l'application telle que définie précédemment, caractérisée en ce que les composés ayant une activité antiminéralocorticoïde répondent à la formule générale (Iⱼ) : 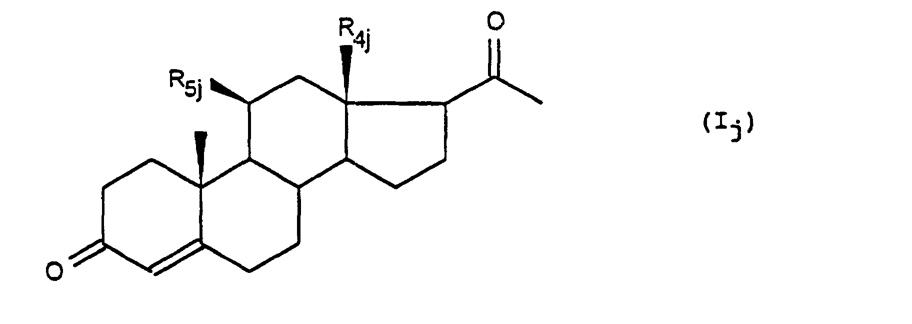 dans laquelle R₄ⱼ est un groupement alkényle ou alkynyle renfermant de 1 à 8 atomes de carbone et R₅ⱼ est un radical hydroxyle ou un atome d'hydrogène.

La présente invention a tout particulièrement pour objet l'application telle que définie précédemment, des produits répondant à la formule générale (Iⱼ) dans laquelle
soit R₄ⱼ et un radical CH₂-CH=CH₂, CH=CH₂, CH₂-C≡CH et R₅ⱼ est un hydrogène,
soit R₄ⱼ est un radical -CH₂-C≡CH et R₅ⱼ est un radical OH.

Les produits de formule (Iⱼ) sont inhibiteurs de la biosynthèse de l'aldostérone. Ils sont décrits dans les publications suivantes :
- A. Viger et al., Tetrahedron (1988) 44 1127, J. Steroïd Biochem. (1988) 30 469,
- B.W. Metcalf et al., Tet. Lett. (1985) 26 1137-1140.

L'invention utilise des compositions pharmaceutiques renfermant comme principe actif au moins un médicament tel que défini ci-dessus, destinées à la prévention ou au traitement des manifestations liées à la dépendance ou au syndrome de sevrage spontané ou précipité, provoqué par les narcotiques ou mélanges de narcotiques.

Les composés utilisés par voie digestive, parentérale ou locale, par exemple par voie percutanée. Ils peuvent être prescrits sous forme de comprimés simples ou dragéifiés, de gélules, de granulés, de suppositoires, d'ovules, de préparation injectables, de pommades, de crèmes, de gels, de microsphères, d'implants, de patchs, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention utilise notamment les compositions pharmaceutiques renfermant comme principe actif au moins un médicament tel que défini ci-dessus, répondant à l'une quelconque des formules générales (I), (Iₐ), (I_{b}), (I_{c}), (I_{d}), (Iₑ), (I_{f}), (I_{g}), (Iₕ), (Iᵢ) ou (Iⱼ), telles que définies précédemment.

L'invention utilise plus particulièrement les compositions pharmaceutiques renfermant comme principe actif au moins un médicament tel que défini ci-dessus, choisis dans la liste suivante :
- γ-lactone de l'acide 10β-éthynyle,17β-hydroxy 3-oxo-19-nor-17α-pregna-4,9(11)-diène-21-carboxylique,
- 17β-hydroxy-3-oxo-7α-propyl-17α-pregn-4-ène-21-carboxylate de potassium,
- γ-lactone de l'acide 11β-allényl-17β-hydroxy-3-oxo-19-nor-17α-pregna-4,9(11)-diène-21-carboxylique,
- γ-lactone de l'acide 17β-hydroxy-7α-méthoxycarbonyle-15β,16β-méthylène-3-oxo-17α-pregn-4-ène-21-carboxylique,
- γ-lactone de l'acide 7α-acétylthio-17β-hydroxy-3-oxo-17α-pregn-4-ène-21-carboxylique,
- γ-lactone de l'acide 17β-hydroxy-7α-méthoxycarbonyle-3-oxo-17α-pregn-4-ène-21-carboxylique,
- γ-lactone de l'acide 17β-hydroxy-3-oxo-17α-pregna-4,6-diène-21-carboxylique,
- 13β-(propèn-2-yl)-18-nor-pregn-4-ène-3,20-dione.

La posologie utile varie en fonction du type de sevrage ou de dépendance à prévenir ou à traiter et de la voie d'administration. Elle peut varier de 1 à 1000 mg par jour chez l'adulte par voie orale.

### ETUDE PHARMACOLOGIQUE

### A) Evaluation du syndrome de sevrage précipité par la naloxone chez la souris.

### 1 - Matériel et méthode.

### 1.1 Animaux.

Les expériences ont été réalisées chez des souris mâles Swiss (Charles River, France) d'un poids corporel compris entre 20 et 25 g. Durant les essais, les animaux ont eu libre accès à la nourriture et à l'eau de boisson. Le nombre d'animaux par groupe est de 10 à 13 souris.

### 1.2 Produits.

Les antiminéralocorticoïdes (AM) suivants :
- AM1 Spironolactone,
- AM2 17β-hydroxy 3-oxo 7α-propyl- (17α)-pregn-4-ène-21-carboxylate de potassium.
ont été mis en suspension dans de la méthyl cellusose à 0,5 % et administrés par voie orale (p.o). La morphine a été mise en solution dans du sérum physiologique et administrée par voie sous-cutanée (s.c). Enfin, le chlorhydrate de naloxone a été solubilisé dans de l'eau distillée et injecté par voie intra-péritonéale.

Tous les produits ont été administrés à l'animal sous un volume de 25 ml/kg.

### 1.3 Modalités de traitement et doses administrées.

### 1.3.1 Traitement par la morphine : induction de l'état de dépendance morphinique.

Le premier jour (J1), les animaux ont reçu 5 injections sous-cutanées avec des doses croissantes de morphine réparties dans la matinée et sur le début de l'après midi, à une heure d'intervalle. Les 3 jours suivants (J2, J3, J4), ces mêmes animaux ont été traités le matin par deux administrations de la dose maximale utilisée au jour 1.

| Morphine - Doses (mg/kg, s.c.) | | | | Dose cumulée (mg/kg) |
|---|---|---|---|---|
| J1 | J2 | J3 | J4 | |
| 8 | 100 | 100 | 100 | 799 |
| 16 | 100 | 100 | 100 | |
| 25 | | | | |
| 50 | | | | |
| 100 | | | | |

### 1.3.2 Traitement répété par les produits étudiés.

Les antiminéralocorticoïdes AM1 et AM2 ont été administrés par voie orale sous forme d'un traitement quotidien effectué 2 heures avant l'administration de morphine et ceci durant 4 jours.

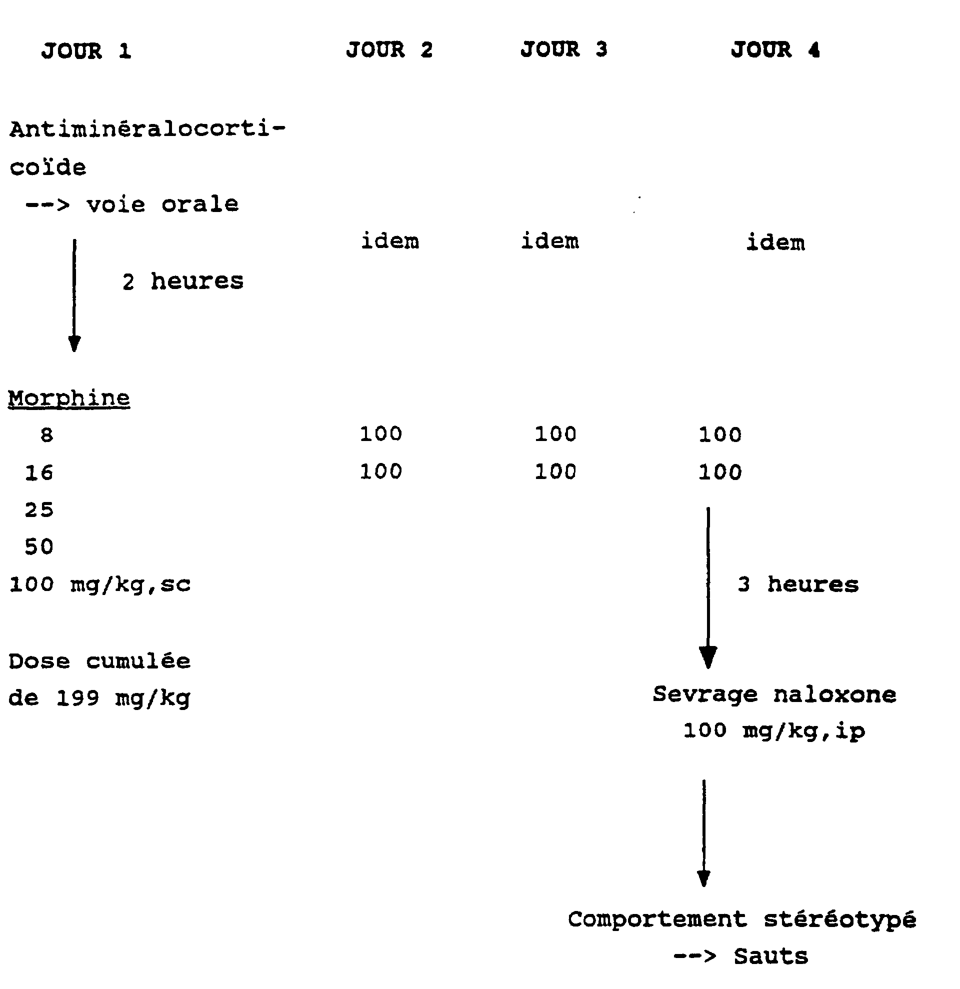

### 1.4 Mode opératoire

La technique utilisée s'inspire de celle décrite par Way et al. J. Pharmacol. Exp. Ther. (1969), 167 : 1-8 et Huidobro et Maggiolo Acta Physiol. Pharmacol. Latinoam. (1961), 11 : 201-9. Ainsi, l'administration réitérée d'un morphinique induit un phénomène de dépendance physique qui peut être facilement mis en évidence par la précipitation d'un syndrome de sevrage à l'aide d'un antagoniste opiacé tel que la naloxone. Ce syndrome se manifeste, chez la souris sous la forme d'un comportement stéréotypé caractérisé par l'apparition de sauts répétitifs dont le nombre est en relation avec la durée et l'intensité du traitement par le morphinique.

Le sevrage des animaux a été réalisé le quatrième jour ; trois heures après le dernier traitement, à l'aide de chlorhydrate de naloxone injecté par voie intrapéritonéale à la dose de 100 mg/kg. Immédiatement après cette injection, les animaux ont été placés individuellement dans des cylindres en plexiglas (hauteur = 40 cm, diamètre = 20 cm). Le nombre de sauts consécutifs au sevrage et effectués par chaque animal a été compté durant une période de 10 minutes.

Les résultats qui figurent dans les tableaux représentent les moyennes des valeurs individuelles assorties de l'erreur standard à la moyenne (m ± esm).

### 2. Résultats.

### 2.1 Sevrage précipité par la naloxone chez des souris normales traitées par la morphine.

| | Doses (mg/kg) | Nombre de sauts/10 minutes (m ± esm) | % protection |
|---|---|---|---|
| Témoins | 0 | 7 ± 5 | |
| | | | |
| Morphine | 799 s.c (cumulée sur 4 jours) | 76 ± 15 | |
| | | | |
| AM1 | 4 x 10 p.o | 3 ± 2 | |
| | | | |
| Morphine + AM1 | 799 s.c + 4 x 10 p.o | 51 ± 7 | - 33 |
| Témoins | 0 | 0 | |
| | | | |
| Morphine | 799 s.c (cumulée sur 4 jours) | 114 ± 8 | |
| | | | |
| AM1 | 4 x 20 p.o | 11 ± 4 | |
| | | | |
| Morphine + AM1 | 799 s.c + 4 x 20 p.o | 77 ± 8** | - 32 |
| Témoins | 0 | 0 | |
| | | | |
| Morphine | 799 s.c (cumulée sur 4 jours) | 99 ± 13 | |
| | | | |
| AM2 | 4 x 20 p.o | 4 ± 4 | |
| | | | |
| Morphine + AM2 | 799 s.c + 4 x 20 p.o | 51 ± 8 | - 48 |
| Témoins | 0 | 7 ± 3 | |
| | | | |
| Morphine | 799 s.c (cumulée sur 4 jours) | 102 ± 10 | |
| | | | |
| AM2 | 4 x 10 p.o | 2 ± 2 | |
| | | | |
| AM2 | 4 x 50 p.o | 3 ± 2 | |
| | | | |
| Morphine + AM2 | 799 s.c + 4 x 10 p.o | 76 ± 16 | - 25 |
| | | | |
| | | | |
| Morphine + AM2 | 799 s.c + 4 x 50 p.o | 61 ± 8** | - 40 |
| n = 10 souris/groupe ; ** p. <0,01 selon le test de Dunnett par rapport au groupe morphine. | | | |

### Discussion - Conclusion

Co-administré durant quatre jours avec la morphine, le composé AM2 (4x20 mg/kg, p.o) inhibe de façon significative (-48 %) le syndrome de sevrage précipité par la naloxone chez des souris dépendantes à la morphine.

En conclusion, ces résultats confirment la participation des glucocorticoïdes endogènes dans le syndrome de sevrage morphinique, tel qu'il se manifeste chez la souris par un comportement stéréotypé de sauts, puisque ce dernier est inhibé par des antiminéralocorticoïdes.

L'activité des antiminéralocorticoïdes, à travers les résultats obtenus avec les composés AM1 et AM2 montre qu'un antagoniste des minéralocorticoïdes peut avoir un effet bénéfique en clinique humaine dans la prévention et traitement du syndrome de sevrage narcotique.

### B) Evaluation de la dépendance induite par la cocaïne

### 1 - Méthode.

Le produit anti-minéralocorticoïde AM2 a été testé sur 5 rats qui ont été entrainés à l'auto-administration de cocaïne pendant des périodes quotidiennes d'une heure.

Ces rats ont été prétraités 1 heure avant le début des sessions d'auto-administration.

Le produit AM2 dissous dans une solution saline a été administré par voie intra-péritonéale. Les doses s'échelonnent de 10 à 100 mg/kg.

### 2 - Résultat.

Le pré-traitement avec 10 ou 20 mg/kg de produit AM2 a peu ou aucun effet sur l'auto-administration de cocaïne.

Cependant, le pré-traitement avec 50 ou 75 mg/kg de produit AM2 entraîne une diminution significative de l'auto-administration de cocaïne.

### 3 - Conclusion.

D'une part ces résultats suggèrent que les récepteurs des minéralocorticoïdes sont impliqués dans le comportement de renforcement à la cocaïne.

D'autre part l'activité des anti-minéralocorticoïdes, à travers les résultats obtenus avec le composé AM2 montre qu'un antagoniste des minéralocorticoïdes peut avoir un effet bénéfique en clinique humaine dans le traitement de la dépendance aux narcotiques et notamment à la cocaïne.

## Revendications

1. Application des composés ayant une activité antiminéralocorticoïde pour la préparation de médicaments destinés à la prévention ou au traitement des manifestations liées à la dépendance ou au syndrome de sevrage spontané ou précipité, provoqué par les narcotiques ou mélanges de narcotiques.

2. Application selon la revendication 1, **caractérisée en ce que** le narcotique est un morphinomimétique choisi parmi l'héroïne, la morphine et la méthadone.

3. Application selon la revendication 1, **caractérisée en ce que** le narcotique est la cocaïne.

4. Application selon l'une quelconque des revendications 1 à 3, **caractérisée en ce que** les composés ayant une activité antiminéralocorticoïde répondent à la formule générale (I) : 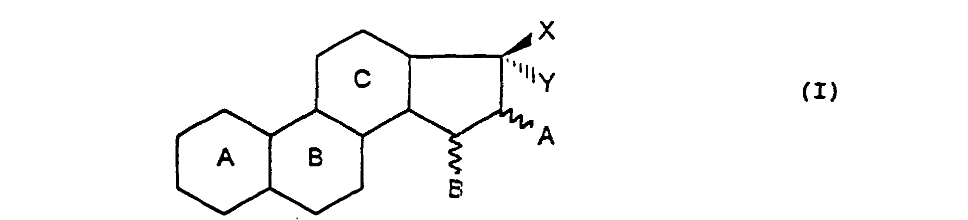 dans laquelle les cycles A, B et C ont l'une des structures suivantes : 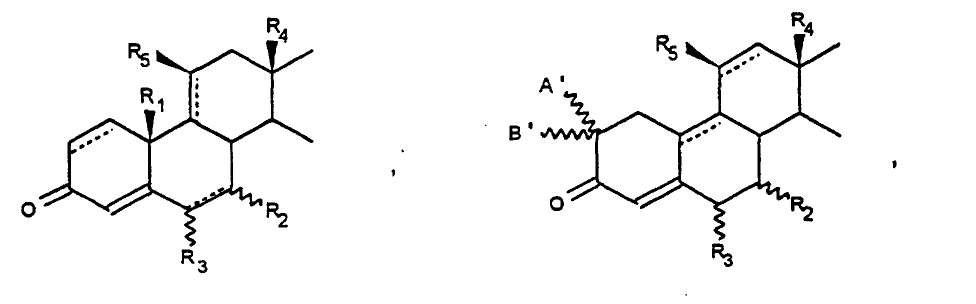 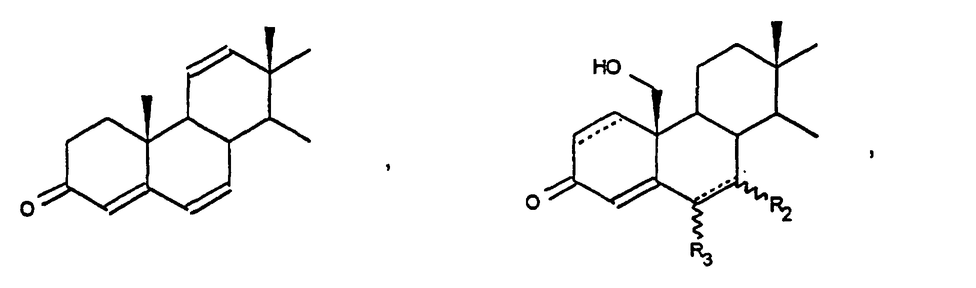 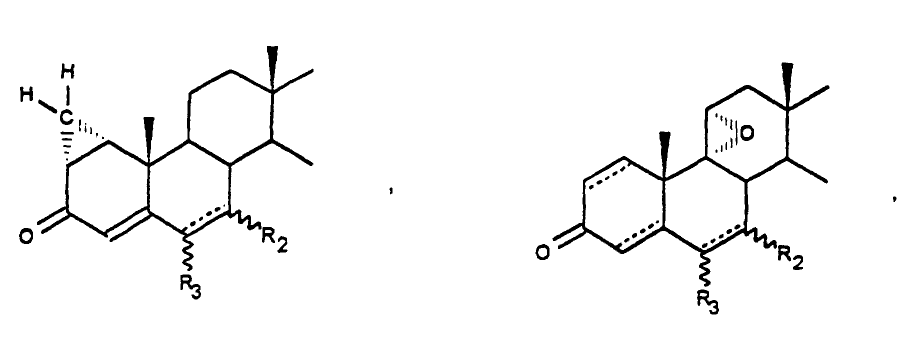 et dans laquelle :
soit X et Y représentent les groupements : 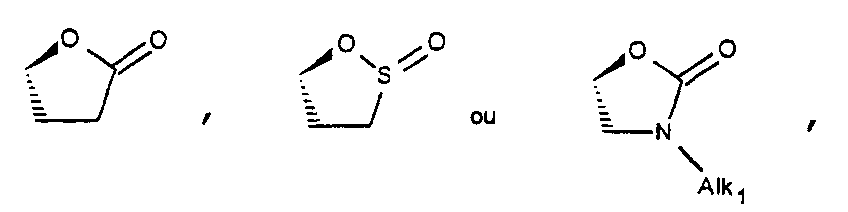 Alk₁ représentant un groupement alkyle renfermant au plus 8 atomes de carbone,
soit X représente un radical hydroxyle, acétyloxy, propionyloxy, méthoxy ou éthoxy et Y représente un radical CH₂CH₂CO₂M, CH₂CH₂SO₂M ou CH₂CH₂CH₂OH, M étant un atome d'hydrogène, un atome de métal alcalin ou un radical ammonium,
soit X représente un radical COCH₂Z, dans lequel Z représente un atome d'hydrogène, un radical hydroxyle ou un radical acyloxy renfermant de 1 à 18 atomes de carbone, et Y représente un atome d'hydrogène,
soit X représente un radical 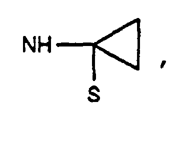 S étant un radical alkyle renfermant au plus 8 atomes de carbone, ou un atome d'hydrogène et Y représente un atome d'hydrogène, A et B sont des atomes d'hydrogène ou forment ensemble un pont méthylénique en position 15α,16α ou 15β,16β, A' et B' sont des atomes d'hydrogène, des radicaux alkyle renfermant de 1 à 4 atomes de carbone, ou forment avec le carbone qui les portent un radical cyclopropyle, R₁ représente un radical méthyle ou un groupement 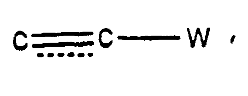 dans lequel W représente
soit un atome d'hydrogène,
soit un radical alkyle renfermant au plus 8 atomes de carbone éventuellement substitué par un radical hydroxyle, carboxy libre, estérifié ou salifié, amino, tritylamino, chloroacétylamino, trifluoroacétylamino, halogène, monoalkylamino, dialkylamino, chaque radical alkyle renfermant au plus 8 atomes de carbone,
soit un radical aryle ou aralkyle renfermant au plus 14 atomes de carbone, éventuellement substitué par un radical hydroxyle, carboxy libre estérifié ou salifié, amino, monoalkylamino, dialkylamino, alkyle, alkoxy ou alkylthio, chaque radical alkyle renfermant au plus 8 atomes de carbone,
soit un atome d'halogène,
soit un radical trialkylsilyle, chaque radical alkyle renfermant au plus 8 atomes de carbone,
R₂ et R₃ sont tels que
soit R₂ et R₃ forment ensemble un pont méthylénique en position 6α,7α ou 6β,7β,
soit R₂ et R₃ sont des atomes d'hydrogène,
soit R₃ est un atome d'hydrogène et R₂ représente un groupement SCOCH₃, CO₂Alk, Alk étant un radical alkyle renfermant au plus 8 atomes de carbone, alkyle, alkényle ou alkynyle renfermant renfermant au plus 8 atomes de carbone et éventuellement substitués par un radical hydroxyle, carboxy libre, estérifié ou salifié, halogène, amino, monoalkylamino, dialkylamino, chaque radical alkyle renfermant au plus 8 atomes de carbone, R₄ représente un radical alkyle, alkényle ou alkynyle renfermant au plus 8 atomes de carbone, R₅ représente soit un radical allényle, soit un radical hydroxyle, soit un atome d'hydrogène, les traits en pointillés représentent une seconde liaison éventuelle, les traits ondulés indiquent que les substituants sont en position α ou β, ainsi que les sels des produits de formule (I) avec les acides et les bases pharmaceutiquement acceptables.

5. Application selon la revendication 4, **caractérisée en ce que** les composés ayant une activité antiminéralocorticoïde répondent à la formule générale (Iₐ) : 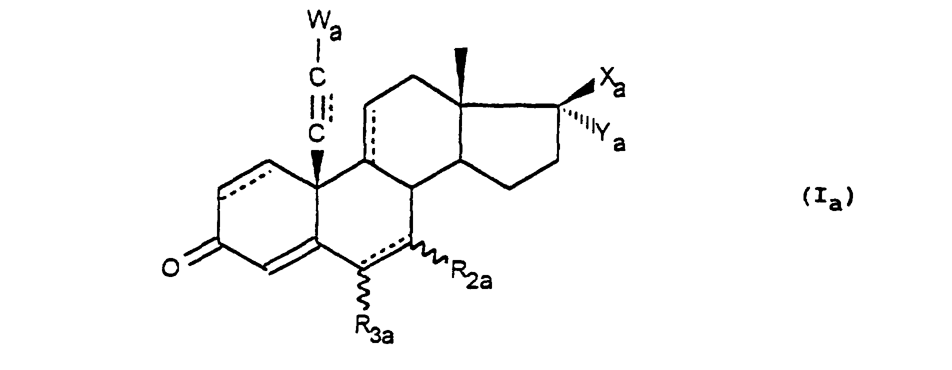 dans laquelle :
soit Xₐ et Yₐ représentent les groupements 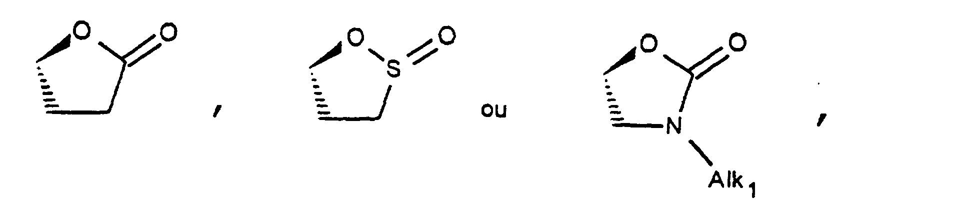 Alk₁ étant tel que défini à la revendication 4,
soit Xₐ représente un radical hydroxyle, acétyloxy, propionyloxy, méthoxy ou éthoxy et Yₐ représente un radical CH₂CH₂CO₂M, CH₂CH₂SO₂M ou CH₂CH₂CH₂OH, M étant tel que défini à la revendication 4,
R₂ₐ, R₃ₐ et Wₐ ont respectivement les même valeurs que R₂, R₃ et W telles que définies à la revendication 4 et les traits pointillés ou ondulés ont la signification indiquée à la revendication 4.

6. Application selon les revendications 4 ou 5, **caractérisée en ce que** les composés répondant aux formules générales (I) ou (Iₐ) sont choisis dans la liste suivante :
- γ-lactone de l'acide 10β-éthynyle,17β-hydroxy 3-oxo-19-nor-17α-pregna-4,9(11)-diène-21-carboxylique,
- γ-lactone de l'acide 17β-tydroxy 3-oxo-10β-(1-propynyle)-19-nor-17α-pregna-4,9(11)-diène-21-carboxylique,
- γ-lactone de l'acide 17β-hydroxy-3-oxo-10β-(1-propynyl)-19-nor-17α-pregn-4-ène-21-carboxylique,
- γ-lactone de l'acide 10β-éthynyle 17β-hydroxy 3-oxo-19-nor-17α-pregn-4-ène-21-carboxylique.

7. Application selon la revendication 4, **caractérisée en ce que** les composés ayant une activité antiminéralocorticoïde répondent à la formule générale (I_{b}) : 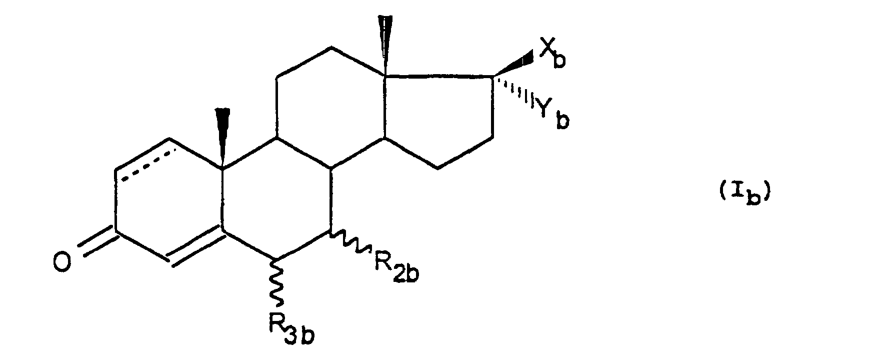 dans laquelle :
soit X_{b} et Y_{b} représentent les groupements 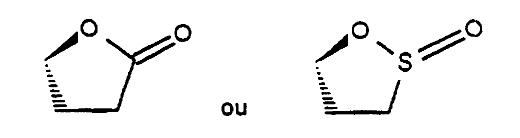
soit X_{b} représente un radical hydroxyle et Y_{b} représente un radical CH₂CH₂CO₂M, M étant tel que défini à la revendication 4,
R_{2b} et R_{3b} sont tels que
ou bien R_{2b} représente un radical alkyle, alkényle ou alkynyle éventuellement substitués tels que définis à la revendication 4 et R_{3b} est un atome d'hydrogène,
ou bien R_{2b} et R_{3b} forment ensemble un pont méthylénique en position 6α,7α ou en position 6β,7β.

8. Application selon l'une des revendications 4 ou 7, **caractérisée en ce que** les composés répondant aux formules générales (I) ou (I_{b}) sont choisis dans la liste suivante :
- (17R)-6β,7β-méthylène-2'-oxydospiro-(androst-4-ène-17,5'-(1',2')-oxathiolane)-3-one
- (17R)-6α,7α-méthylène-2'-oxydospiro-(androst-4-ène-17,5'-(1',2')-oxathiolane)-3-one
- (17R)-7α-méthyl-2'-oxydospiro-(androst-4-ène-17,5'-(1',2')-oxathiolane)-3-one
- (17R)-7α-n-propyl-2'-oxydospiro-(androst-4-ène-17,5'-(1',2')-oxathiolane)-3-one
- γ-lactone de l'acide 17β-hydroxy-3-oxo-7α-propyl-17α-pregna-1,4-diène-21-carboxylique,
- 17β-hydroxy-3-oxo-7α-propyl-17α-pregna-1,4-diène-21-carboxylate de potassium,
- γ-lactone de l'acide 17β-hydroxy-3-oxo-7α-propyl-(17α)-prégn-4-ène-21-carboxylique,
- 17β-hydroxy-3-oxo-7α-propyl-(17α)-pregn-4-ène-21-carboxylate de potassium.

9. Application selon la revendication 4, **caractérisée en ce que** les composés ayant une activité antiminéralocorticoïde, répondant à la formule générale (I_{c}) : 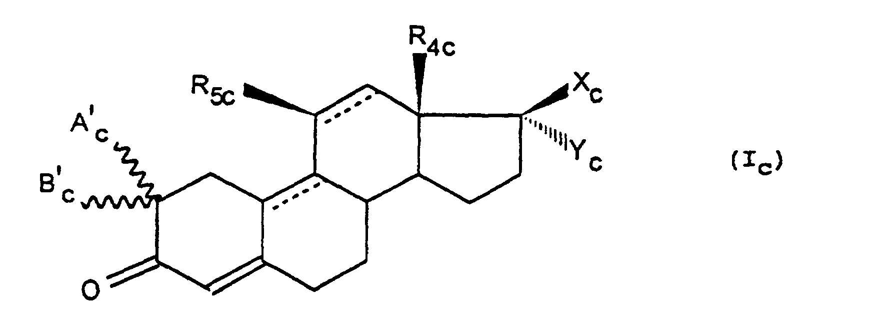 dans laquelle
soit X_{c} et Y_{c} représentent un groupement 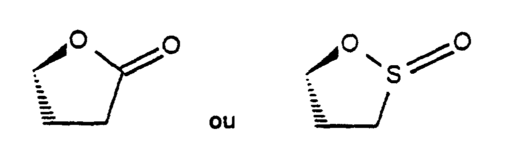
soit X_{c} représente un radical hydroxyle et Y_{c} représente un groupement CH₂CH₂CO₂M ou CH₂CH₂SO₂M, M étant tel que défini à la revendication 4.
soit X_{c} représente un radical COCH₂Z dans lequel Z est tel que défini à la revendication 4 et Y_{c} est un atome d'hydrogène,
A'_{c} et B'_{c} ont respectivement les mêmes valeurs que A' et B' telles que définies à la revendication 4,
R_{4c} est un radical méthyle ou éthyle, R_{5c} est soit un atome d'hydrogène, soit un radical allényle, étant entendu que lorsque R_{5c} est un radical allényle A'_{c} et B'_{c} sont des atomes d'hydrogène, R_{4c} est un radical méthyle, X_{c} et Y_{c} forment ensemble un groupement 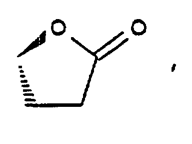 les traits en pointillés en position 9-10 forment une seconde liaison, et ceux en position 11-12 ne forment pas une seconde liaison.

10. Application selon l'une des revendications 4 ou 9, **caractérisée en ce que** les composés répondant aux formules générales (I) ou (I_{c}) sont choisis dans la liste suivante :
- 2,2-diméthyl 19-nor pregn-4-ène-3,20-dione,
- 21-acétoxy 2,2-diméthyl 19-nor-pregn-4-ène 3,20-dione,
- 2,2-diméthyl 21-hydroxy 19-nor-pregn-4-ène 3,20-dione,
- 2,2-diméthyl 19-nor-pregna-4,9-diène 3,20-dione,
- 21-acétoxy 2,2-diméthyl 19-nor-pregna-4,9-diène 3,20-dione,
- 2,2-diméthyl 21-hydroxy 19-nor-pregna-4,9-diène 3,20-dione,
- 2,2-diméthyl 19-nor-pregna-4,9,11-triène 3,20-dione,
- 21-acétoxy 2,2-diméthyl 19-nor-pregna-4,9,11-triène 3,20-dione,
- 2,2-diméthyl 21-hydroxy 19-nor-pregna-4,9,11-triène 3,20-dione,
- (17R) 2'-oxydospiro-(estra-4,9-diène-17,5'-(1',2')-oxathiolane) 3-one,
- (17R) 2'-oxydospiro-(estra-4,9,11-triène-17,5'-(1',2')-oxathiolane) 3-one,
- (17R) 11β-hydroxy 2'-oxydospiro-(estra-4,9-diène-17,5'-(1',2')-oxathiolane) 3-one,
- 2,2-diméthyl-13-éthyl-21-hydroxy-18,19-dinor-pregn-4-ène 3,20-dione,
- γ-lactone de l'acide 11β-allényl-17β-hydroxy-3-oxo-19-nor-17α-pregna-4,9-diène-21-carboxylique.

11. Application selon la revendication 4, **caractérisé en ce que** les composés ayant une activité antiminéralocorticoïde répondent à la formule générale (I_{d}) : 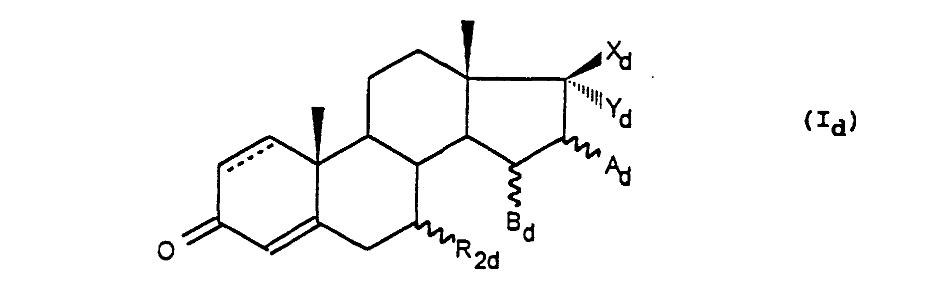 dans laquelle :
soit X_{d} et Y_{d} représentent le groupement 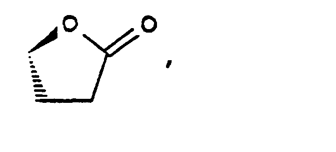
soit X_{d} représente un radical hydroxyle et Y_{d} représente un radical CH₂CH₂CO₂M, M étant tel que défini à la revendication 4,
A_{d} et B_{d} ont respectivement les mêmes valeurs que A et B telles que définies à la revendication 4,
R_{2d} représente soit un radical thioacétyle, soit un radical CO₂Alk, Alk étant un radical alkyle renfermant au plus 8 atomes de carbone et le trait pointillé en position 1-2, représentant une éventuelle double liaison.

12. Application selon l'une des revendications 4 ou 11, **caractérisée en ce que** les composés répondant à la formule générale (I) ou (I_{d}) sont choisis dans la liste suivante :
- γ-lactone de l'acide 7α-acétylthio-17β-hydroxy-15β,16β-méthylène-3-oxo-17α-pregna-1,4-diène-21-carboxylique,
- γ-lactone de l'acide 17β-hydroxy-7α-méthoxycarbonyle-15β,16β-méthylène-3-oxo-17α-pregn-4-ène-21-carboxylique,
- γ-lactone de l'acide 7α-acétylthio-17β-hydroxy-3-oxo-17α-pregn-4-ène-21-carboxylique,
- γ-lactone de l'acide 17β-hydroxy-7α-méthoxycarbonyle-3-oxo-17α-pregn-4-ène-21-carboxylique,
- sel de potassium de l'acide 17β-hydroxy-7α-méthoxycarbonyle-3-oxo-pregn-4-ène-21-carboxylique.

13. Application selon la revendication 4, **caractérisée en ce que** les composés ayant une activité antiminéralocorticoïde répondent à la formule générale (Iₑ) : 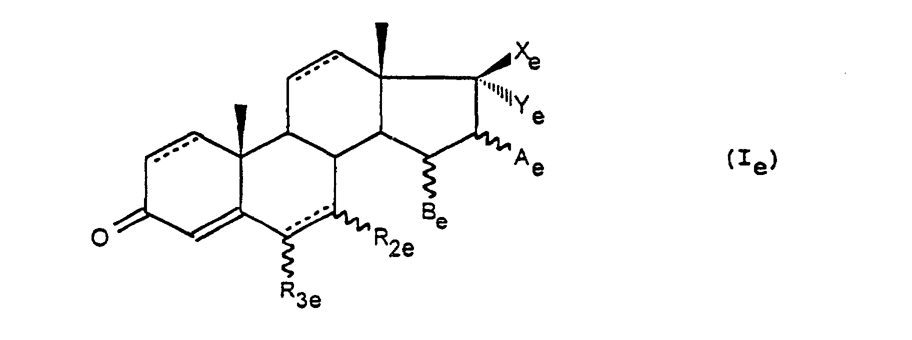 dans laquelle :
soit Xₑ et Yₑ représentent le groupement 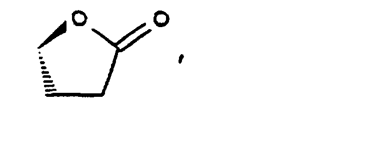
soit Xₑ représente un radical hydroxyle et Yₑ représente un radical CH₂CH₂CO₂M, M étant tel que défini précédemment,
R₂ₑ ou R₃ₑ sont tels que :
ou bien R₂ₑ et R₃ₑ forment ensemble un pont méthylénique en position 6α,7α ou 6β,7β,
ou bien R₂ₑ et R₃ₑ sont des atomes d'hydrogène,
Ae et Bₑ ont respectivement les mêmes valeurs que A et B telles que définies à la revendication 4 et les traits pointillés ou ondulés gardent la même signification qu'à la revendication 4.

14. Application selon l'une des revendications 4 ou 11, **caractérisée en ce que** les composés répondant à la formule générale (I) ou (Iₑ) sont choisis dans la liste suivante :
- γ-lactone de l'acide 17β-hydroxy-6β,7β,15β,16β-diméthylène-3-oxo-17α-pregna-1,4-diène-21-carboxylique,
- γ-lactone de l'acide 17β-hydroxy-6β,7β,15β,16β-diméthylène-3-oxo-17α-pregna-4-ène-21-carboxylique,
- γ-lactone de l'acide 17β-hydroxy-3-oxo-17α-pregna-4,6-diène-21-carboxylique,
- sel de potassium de l'acide 17β-hydroxy-3-oxo-17α-pregna-4,6-diène-21-carboxylique,
- γ-lactone de l'acide 17β-hydroxy-3-oxo-17α-pregna-4,6,11-triène-21-carboxylique,
- γ-lactone de l'acide 17β-hydroxy-6β,7β-méthylène-3-oxo-17α-pregn-4-ène-21-carboxylique,
- sel de potassium de l'acide 17β-hydroxy-6β,7β-méthylène-3-oxo-17α-pregn-4-ène-21-carboxylique.

15. Application selon la revendication 4, **caractérisée en ce que** les composés ayant une activité antiminéralocorticoïde répondent à la formule générale (I_{f}) : 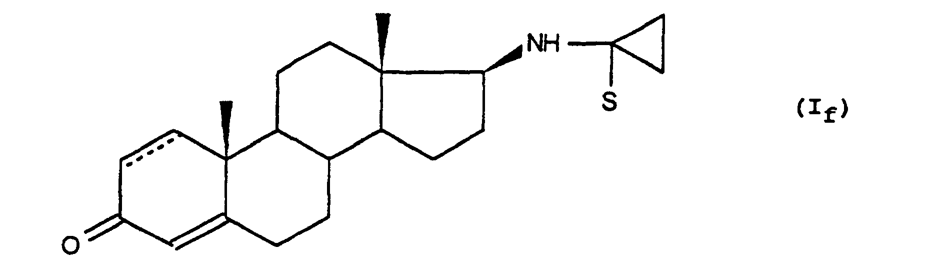 dans laquelle S représente un radical alkyle renfermant de 1 à 8 atomes de carbone, ou un atome d'hydrogène.

16. Application selon la revendication 4, **caractérisée en ce que** les composés ayant une activité antiminéralocorticoïde répondent à la formule générale (I_{g}) : 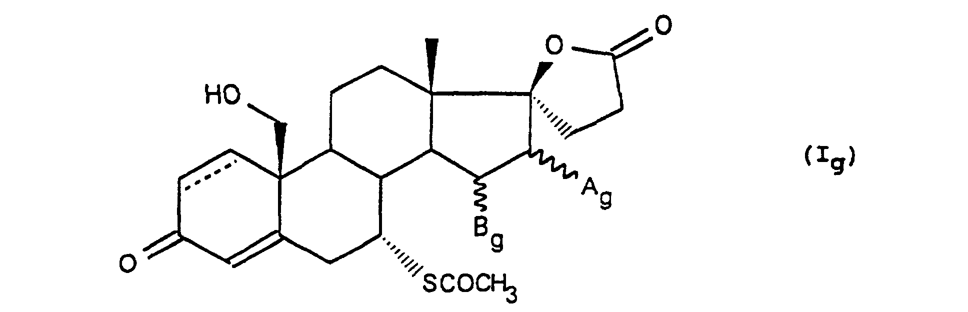 dans laquelle A_{g} et B_{g} ont respectivement les mêmes valeurs que A et B telles que définies à la revendication 4.

17. Application selon la revendication 4, **caractérisée en ce que** les composés ayant une activité antiminéralocorticoïde répondant à la formule générale (Iₕ) : 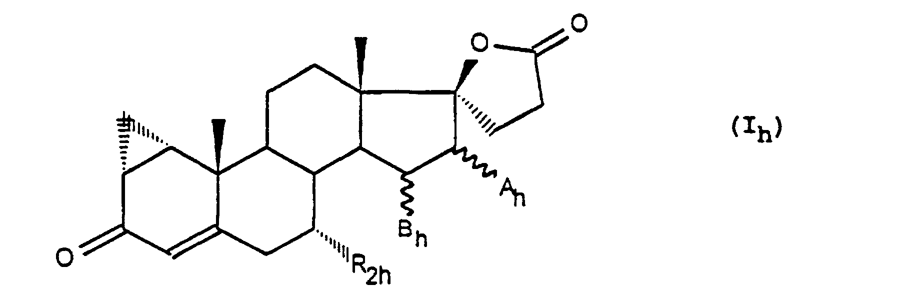 dans laquelle Aₕ, Bₕ et R₂ₕ ont respectivement les mêmes valeurs que A, B et R₂ telles que définies à la revendication 4.

18. Application selon la revendication 4, **caractérisée en ce que** les composés ayant une activité antiminéralocorticoïde répondent à la formule générale (Iᵢ) : 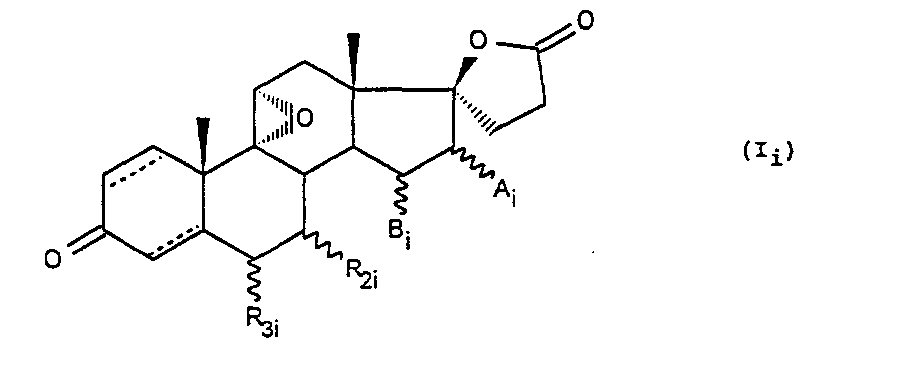 dans laquelle Aᵢ, Bᵢ, R₂ᵢ et R₃ᵢ ont respectivement les mêmes valeurs que A, B, R₂ et R₃ telles que définies à la revendication 4.

19. Application selon la revendication 4, **caractérisée en ce que** les composés ayant une activité antiminéralocorticoïde répondent à la formule générale (Iⱼ) : 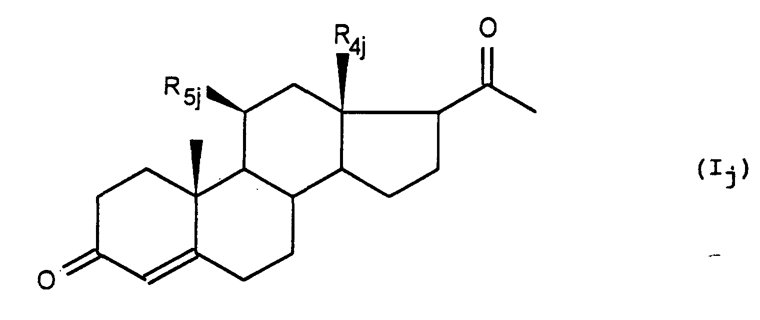 dans laquelle R₄ⱼ est un groupement alkényle ou alkynyle renfermant de 1 à 8 atomes de carbone et R₅ⱼ est un radical hydroxyle ou un atome d'hydrogène.

## Patentansprüche

1. Verwendung von Verbindungen mit einer antimineralcorticoiden Aktivität zur Herstellung von Arzneimitteln, die für die Vorbeugung oder die Behandlung von mit der Abhängigkeit oder dem spontanen oder beschleunigten Entzugssyndrom verbundenen Erscheinungen vorgesehen sind, hervorgerufen durch Narkotika oder Mischungen von Narkotika.

2. Verwendung nach Anspruch 1, **dadurch gekennzeichnet, daß** das Narkotikum ein morphinomimetisches Narkotikum ist, ausgewählt unter Heroin, Morphin und Methadon.

3. Verwendung nach Anspruch 1, **dadurch gekennzeichnet, daß** das Narkotikum Kokain ist.

4. Verwendung nach irgendeinem der Ansprüche 1 bis 3, **dadurch gekennzeichnet, daß** die Verbindungen mit einer antimineralcorticoiden Aktivität der allgemeinen Formel (I) 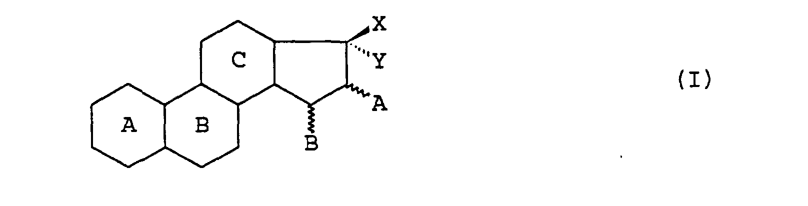 entsprechen, in der die Ringe A, B und C eine der folgenden Strukturen besitzen: 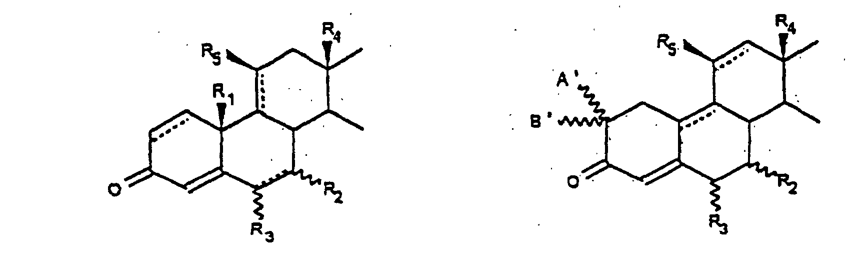 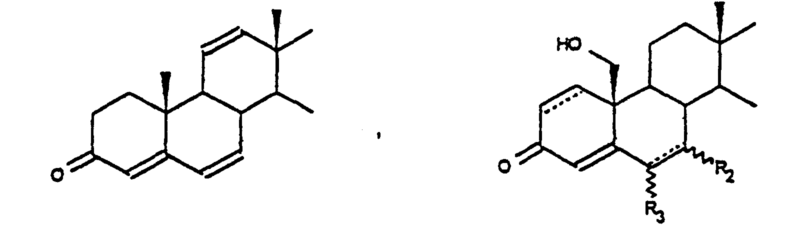 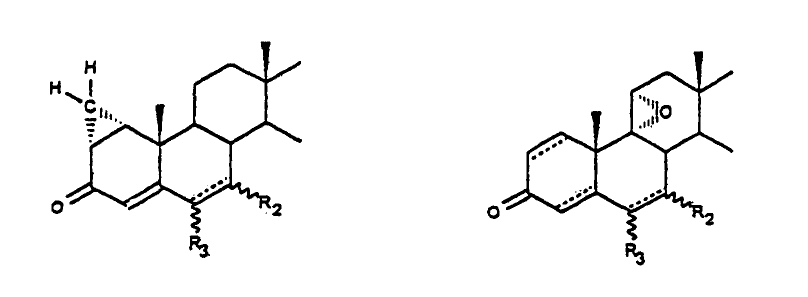 und in der
entweder
X und Y die Gruppen 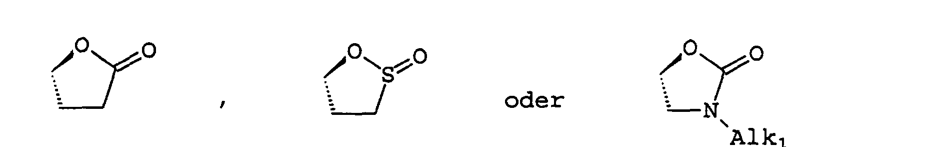 darstellen, worin
Alk₁ eine Gruppe Alkyl mit höchstens 8 Kohlenstoffatomen ist,
oder X einen Rest Hydroxyl, Acetyloxy, Propionyloxy, Methoxy oder Ethoxy bedeutet und Y einen Rest CH₂CH₂CO₂M, CH₂CH₂SO₂M oder CH₂CH₂CH₂OH darstellt und M ein Wasserstoffatom, ein Alkalimetallatom oder ein Ammoniumrest ist,
oder X einen Rest COCH₂Z bedeutet, worin Z ein Wasserstoffatom, ein Rest Hydroxyl oder ein Rest Acyloxy mit 1 bis 18 Kohlenstoffatomen ist und Y ein Wasserstoffatom darstellt,
oder X einen Rest 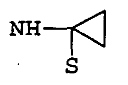 bedeutet, S ein Rest Alkyl mit hächstens 8 Kohlenstoffatomen oder ein Wasserstoffatom ist und
Y ein Wasserstoffatom darstellt,
A und B Wasserstoffatome sind oder zusammen eine methylenische Brücke in Position 15α,16α oder in Position 15β,16β bilden,
A' und B' Wasserstoffatome oder Reste Alkyl mit 1 bis 4 Kohlenstoffatomen sind oder zusammen mit dem Kohlenstoff, der sie trägt, einen Rest Cyclopropyl bilden, R₁ einen Rest Methyl oder eine Gruppe C=C-W darstellt, worin W
entweder ein Wasserstoffatom ist,
oder einen Rest Alkyl mit höchstens 8 Kohlenstoffatomen bedeutet, gegebenenfalls substituiert durch einen Rest Hydroxyl, Carboxy, frei, verestert oder in Salz überführt, Amino, Tritylamino, Chloracetylamino, Trifluoracetylamino, Halogen, Monoalkylamino, Dialkylamino, wobei jeder Rest Alkyl höchstens 8 Kohlenstoffatome umfaßt,
oder einen Rest Aryl oder Aralkyl mit höchstens 14 Kohlenstoffatomen darstellt, gegebenenfalls substituiert durch einen Rest Hydroxyl, Carboxy, frei, verestert oder in Salz überführt, Amino, Monoalkylamino, Dialkylamino, Alkyl, Alkoxy oder Alkylthio, wobei jeder Rest Alkyl höchstens 8 Kohlenstoffatome umfaßt,
oder ein Halogenatom ist,
oder einen Rest Trialkylsilyl bedeutet, wobei jeder Rest Alkyl höchstens 8 Kohlenstoffatome umfaßt,
R₂ und R₃ so beschaffen sind, daß
entweder R₂ und R₃ zusammen eine methylenische Brücke in Position 6α,7α oder in Position 6β,7β bilden,
oder R₂ und R₃ Wasserstoffatome sind,
oder R₃ ein Wasserstoffatom ist und R₂ eine Gruppe SCOCH₃, CO₂Alk, worin Alk ein Rest Alkyl mit höchstens 8 Kohlenstoffatomen ist, Alkyl, Alkenyl oder Alkinyl mit höchstens 8 Kohlenstoffatomen und gegebenenfalls substituiert durch einen Rest Hydroxyl, Carboxy, frei, verestert oder in Salz überführt, Halogen, Amino, Monoalkylamino, Dialkylamino, wobei jeder Rest Alkyl höchstens 8 Kohlenstoffatome umfaßt, darstellt, R₄ einen Rest Alkyl, Alkenyl oder Alkinyl mit höchstens 8 Kohlenstoffatomen bedeutet, R₅ entweder ein Rest Allenyl oder ein Rest Hydroxyl oder ein Wasserstoffatom ist, wobei die punktierten Linien eine eventuelle zweite Bindung darstellen und die Wellenlinien anzeigen, daß sich die Substituenten in Position α oder β befinden,
sowie die Salze der Produkte der Formel (I) mit pharmazeutisch akzeptablen Säuren und Basen.

5. Verwendung nach Anspruch 4, **dadurch gekennzeichnet, daß** die Verbindungen mit einer antimineralcorticoiden Aktivität der allgemeinen Formel (Iₐ) 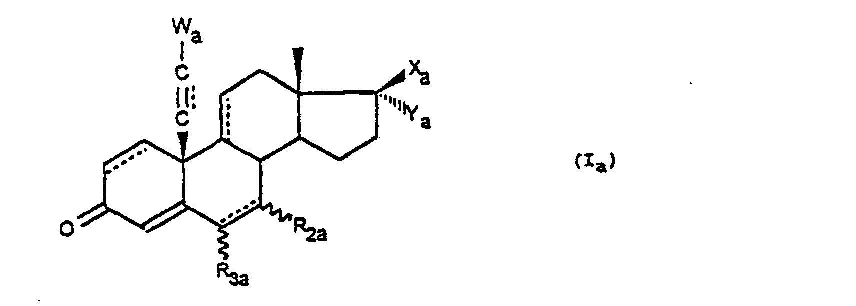 entsprechen, in der
entweder Xₐ und Yₐ die Gruppen 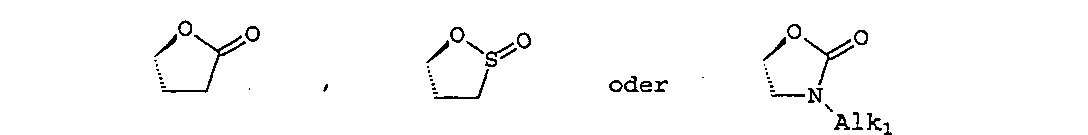 darstellen, worin
Alk₁ wie in Anspruch 4 definiert ist,
oder Xₐ einen Rest Hydroxyl, Acetyloxy, Propionyloxy, Methoxy oder Ethoxy bedeutet und Yₐ einen Rest CH₂CH₂CO₂M, CH₂CH₂SO₂M oder CH₂CH₂CH₂OH darstellt und M wie in Anspruch 4 definiert ist,
R₂ₐ, R₃ₐ und Wₐ jeweils die gleichen Werte wie R₂, R₃ und W besitzen, wie sie in Anspruch 4 definiert sind und die punktierten Linien und die Wellenlinien die in Anspruch 4 angegebenen Bedeutungen besitzen.

6. Verwendung nach Anspruch 4 oder 5, **dadurch gekennzeichnet, daß** die Verbindungen, die den allgemeinen Formeln (I) oder (Iₐ) entsprechen, aus der folgenden Liste gewählt werden:
- γ-Lacton von 10β-Ethinyl-17ß-hydroxy-3-oxo-19-nor-17α-pregna-4,9(11)-dien-21-carbonsäure,
- γ-Lacton von 17β-Hydroxy-3-oxo-10β-(1-propinyl)-19-nor-17α-pregna-4,9(11)-dien-21-carbonsäure,
- γ-Lacton von 17β-Hydroxy-3-oxo-10β-(1-propinyl)-19-nor-17α-pregn-4-en-21-carbonsäure,
- γ-Lacton von 10β-Ethinyl-17β-hydroxy-3-oxo-19-nor-17α-pregn-4-en-21-carbonsäure.

7. Verwendung nach Anspruch 4, **dadurch gekennzeichnet, daß** die Verbindungen mit einer antimineralcorticoiden Aktivität der allgemeinen Formel (I_{b}) 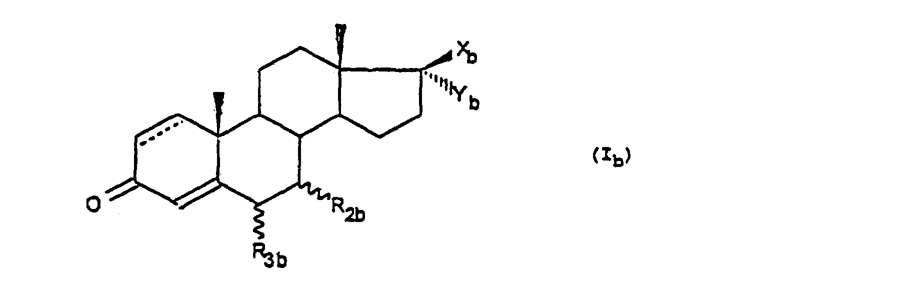 entsprechen, in der
entweder X_{b} und Y_{b} die Gruppen 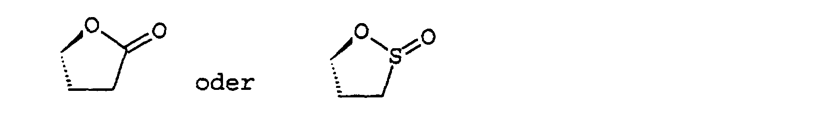 darstellen,
oder X_{b} einen Rest Hydroxyl bedeutet und Y_{b} einen Rest CH₂CH₂CO₂M darstellt, worin M wie in Anspruch 4 definiert ist,
R_{2b} und R_{3b} so beschaffen sind, daß
entweder R_{2b} einen Rest Alkyl, Alkenyl oder Alkinyl bedeutet, gegebenenfalls substituiert wie in Anspruch 4 definiert, und R_{3b} ein Wasserstoffatom ist,
oder R_{2b} und R_{3b} zusammen eine methylenische Brücke in Position 6α,7α oder in Position 6β,7β bilden.

8. Verwendung nach einem der Ansprüche 4 oder 7, **dadurch gekennzeichnet, daß** die Verbindungen, die den allgemeinen Formeln (I) oder (I_{b}) entsprechen, aus der folgenden Liste gewählt werden:
- (17R)-6β,7β-Methylen-2'-oxydospiro-[androst-4-en-17,5'-(1',2')-oxathiolan]-3-on,
- (17R)-6α,7α-Methylen-2'-oxydospiro-[androst-4-en-17,5'-(1',2')-oxathiolan]-3-on,
- (17R)-7α-Methyl-2'-oxydospiro-[androst-4-en-17,5'-(1',2')-oxathiolan]-3-on,
- (17R)-7α-n-Propyl-2'-oxydospiro-[androst-4-en-17,5'-(1',2')-oxathiolan]-3-on,
- γ-Lacton von 17β-Hydroxy-3-oxo-7α-propyl-17α-pregna-1,4-dien-21-carbonsäure,
- 17ß-Hydroxy-3-oxo-7α-propyl-17α-pregna-1,4-dien-21-Kaliumcarboxylat,
- γ-Lacton von 17β-Hydroxy-3-oxo-7α-propyl-(17α)-pregn-4-en-21-carbonsäure,
- 17ß-Hydroxy-3-oxo-7α-propyl-(17α)-pregn-4-en-21-Kalium-carboxylat.

9. Verwendung nach Anspruch 4, **dadurch gekennzeichnet, daß** die Verbindungen mit einer antimineralcorticoiden Aktivität der allgemeinen Formel (I_{c}) 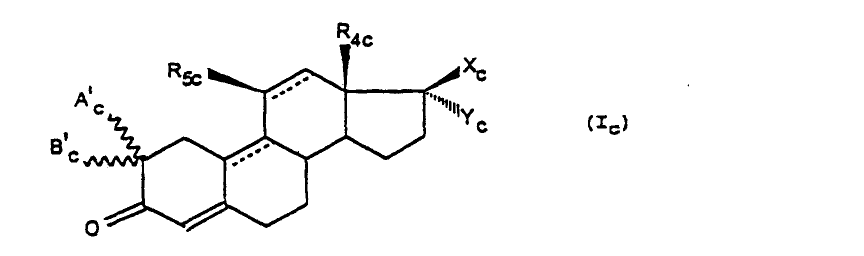 entsprechen, in der
entweder X_{c} und Y_{c} die Gruppen 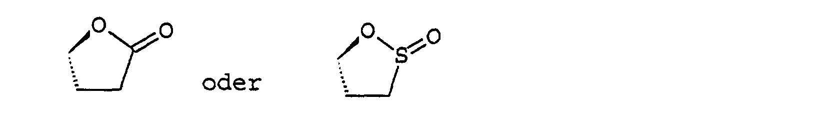 darstellen,
oder X_{c} einen Rest Hydroxyl bedeutet und Y_{c} eine Gruppe CH₂CH₂CO₂M oder CH₂CH₂SO₂M darstellt, worin M wie in Anspruch 4 definiert ist,
oder X_{c} einen Rest COCH₂Z bedeutet, worin Z wie in Anspruch 4 definiert ist und Y_{c} ein Wasserstoffatom darstellt,
A'_{c} und B'_{c} jeweils die gleichen Werte besitzen wie A' und B', wie sie in Anspruch 4 definiert sind,
R_{4c} ein Rest Methyl oder Ethyl ist, R_{5c} entweder ein Wasserstoffatom oder einen Rest Allenyl darstellt,
mit der Maßgabe, daß in dem Fall, wo R_{5c} ein Rest Allenyl ist,
A'_{c} und B'_{c} Wasserstoffatome sind, R_{4c} einen Rest Methyl darstellt, X_{c} und Y_{c} zusammen eine Gruppe 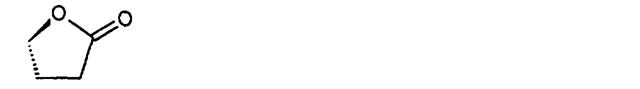 bilden, die punktierten Linien in Position 9-10 eine zweite Bindung bilden und diejenigen in Position 11-12 keine zweite Bindung bilden.

10. Verwendung nach einem der Ansprüche 4 oder 9, **dadurch gekennzeichnet, daß** die Verbindungen, die den allgemeinen Formeln (I) oder (I_{c}) entsprechen, aus der folgenden Liste gewählt werden:
- 2,2-Dimethyl-19-nor-pregn-4-en-3,20-dion,
- 21-Acetoxy-2,2-dimethyl-19-nor-pregn-4-en-3,20-dion,
- 2,2-Dimethyl-21-hydroxy-19-nor-pregn-4-en-3,20-dion,
- 2,2-Dimethyl-19-nor-pregna-4,9-dien-3,20-dion,
- 21-Acetoxy-2,2-dimethyl-19-nor-pregna-4,9-dien-3,20-dion,
- 2,2-Dimethyl-21-hydroxy-19-nor-pregna-4,9-dien-3,20-dion,
- 2,2-Dimethyl-19-nor-pregna-4,9,11-trien-3,20-dion,
- 21-Acetoxy-2,2-dimethyl-19-nor-pregna-4,9,11-trien-3,20-dion,
- 2,2-Dimethyl-21-hydroxy-19-nor-pregna-4,9,11-trien-3,20-dion,
- (17R)-2'-Oxydospiro-[estra-4,9-dien-17,5'-(1',2')-oxathiolan]-3-on,
- (17R)-2'-Oxydospiro[estra-4,9,11-trien-17,5'-(1',2')-oxathiolan]-3-on,
- (17R)-11β-Hydroxy-2'-oxydospiro-[estra-4,9-dien-17,5'-(1',2')-oxathiolan] -3-on,
- 2,2-Dimethyl-13-ethyl-21-hydroxy-18,19-dinor-pregn-4-en-3,20-dion,
- γ-Lacton von 11β-Allenyl-17β-hydroxy-3-oxo-19-nor-17α-pregna-4,9-dien-21-carbonsäure.

11. Verwendung nach Anspruch 4, **dadurch gekennzeichnet, daß** die Verbindungen mit einer antimineralcorticoiden Aktivität der allgemeinen Formel (I_{d}) 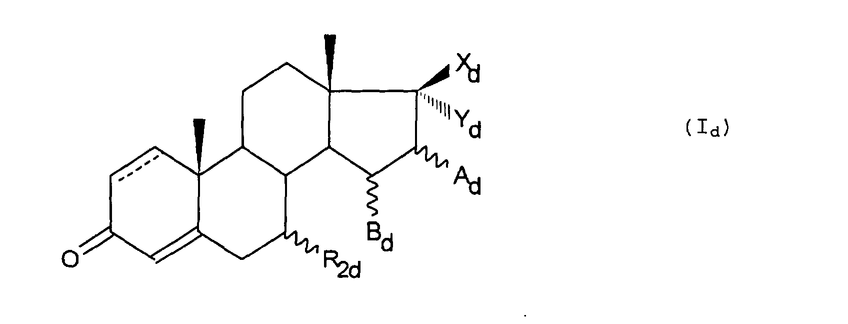 entsprechen, in der
entweder X_{d} und Y_{d} die Gruppe 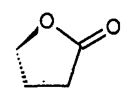 darstellen,
oder X_{d} einen Rest Hydroxyl bedeutet und Y_{d} einen Rest CH₂CH₂CO₂M darstellt, worin M wie in Anspruch 4 definiert ist,
A_{d} und B_{d} jeweils die gleichen Werte besitzen wie A und B, wie sie in Anspruch 4 definiert sind,
R_{2d} entweder einen Rest Thioacetyl oder einen Rest CO₂Alk bedeutet, worin Alk einen Rest Alkyl mit höchstens 8 Kohlenstoffatomen darstellt, und die punktierte Linie in Position 1-2 eine eventuelle Doppelbindung bedeutet.

12. Verwendung nach einem der Ansprüche 4 oder 11, **dadurch gekennzeichnet, daß** die Verbindungen, die den allgemeinen Formeln (I) oder (I_{d}) entsprechen, aus der folgenden Liste gewählt werden:
- γ-Lacton von 7α-Acetylthio-17β-hydroxy-15β,16β-methylen-3-oxo-17α-pregna-1,4-dien-21-carbonsäure,
- γ-Lacton von 17ß-Hydroxy-7α-methoxycarbonyl-15β,16β-methylen-3-oxo-17α-pregn-4-en-21-carbonsäure,
- γ-Lacton von 7α-Acetylthio-17β-hydroxy-3-oxo-17α-pregn-4-en-21-carbonsäure,
- γ-Lacton von 17ß-Hydroxy-7α-methoxycarbonyl-3-oxo-17α-pregn-4-en-21-carbonsäure,
- Kaliumsalz von 17ß-Hydroxy-7α-methoxycarbonyl-3-oxo-pregn-4-en-21-carbonsäure.

13. Verwendung nach Anspruch 4, **dadurch gekennzeichnet, daß** die Verbindungen mit einer antimineralcorticoiden Aktivität der allgemeinen Formel (Iₑ) 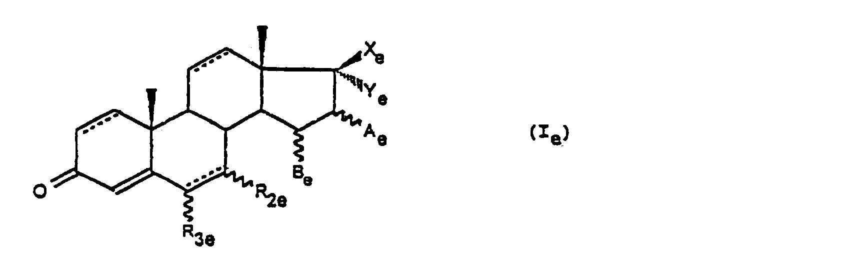 entsprechen, in der
entweder Xₑ und Yₑ die Gruppe 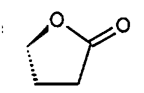 darstellen,
oder Xₑ einen Rest Hydroxyl bedeutet und Yₑ einen Rest CH₂CH₂CO₂M darstellt, worin M wie vorstehend definiert ist,
R₂ₑ und R₃ₑ so beschaffen sind, daß
entweder R₂ₑ und R₃ₑ zusammen eine methylenische Brücke in Position 6α,7α oder in Position 6β,7β bilden,
oder R₂ₑ und R₃ₑ Wasserstoffatome sind,
Aₑ und Bₑ jeweils die gleichen Werte besitzen wie A und B, wie sie in Anspruch 4 definiert sind,
und die punktierten Linien oder Wellenlinien die gleiche Bedeutung behalten wie sie in Anspruch 4 angegeben ist.

14. Verwendung nach einem der Ansprüche 4 oder 11, **dadurch gekennzeichnet, daß** die Verbindungen, die den allgemeinen Formeln (I) oder (Iₑ) entsprechen, aus der folgenden Liste gewählt werden:
- γ-Lacton von 17β-Hydroxy-6β,7β,15β,16β-dimethylen-3-oxo-17α-pregna-1,4-dien-21-carbonsäure,
- γ-Lacton von 17β-Hydroxy-6β,7β,15β,16β-dimethylen-3-oxo-17α-pregn-4-en-21-carbonsäure,
- γ-Lacton von 17β-Hydroxy-3-oxo-17α-pregna-4,6-dien-21-carbonsäure,
- Kaliumsalz von 17β-Hydroxy-3-oxo-17α-pregna-4,6-dien-21-carbonsäure,
- γ-Lacton von 17β-Hydroxy-3-oxo-17α-pregna-4,6,11-trien-21-carbonsäure,
- γ-Lacton von 17β-Hydroxy-6β,7β-methylen-3-oxo-17α-pregn-4-en-21-carbonsäure,
- Kaliumsalz von 17β-Hydroxy-6β,7β-methylen-3-oxo-17α-pregn-4-en-21-carbonsäure.

15. Verwendung nach Anspruch 4, **dadurch gekennzeichnet, daß** die Verbindungen mit einer antimineralcorticoiden Aktivität der allgemeinen Formel (I_{f}) 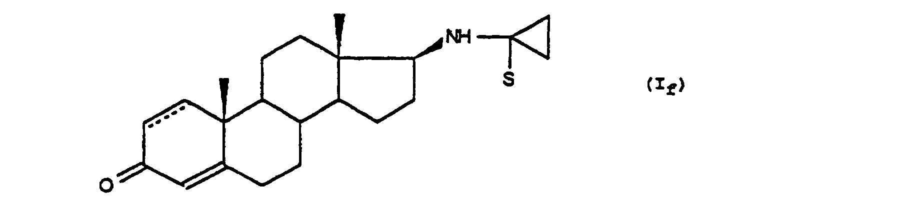 entsprechen, in der S einen Rest Alkyl mit 1 bis 8 Kohlenstoffatomen oder ein Wasserstoffatom darstellt.

16. Verwendung nach Anspruch 4, **dadurch gekennzeichnet, daß** die Verbindungen mit einer antimineralcorticoiden Aktivität der allgemeinen Formel (I_{g}) 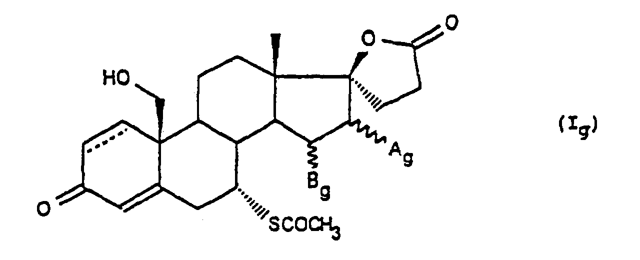 entsprechen, in der A_{g} und B_{g} jeweils die gleichen Werte besitzen wie A und B, wie sie in Anspruch 4 definiert sind.

17. Verwendung nach Anspruch 4, **dadurch gekennzeichnet, daß** die Verbindungen mit einer antimineralcorticoiden Aktivität der allgemeinen Formel (Iₕ) 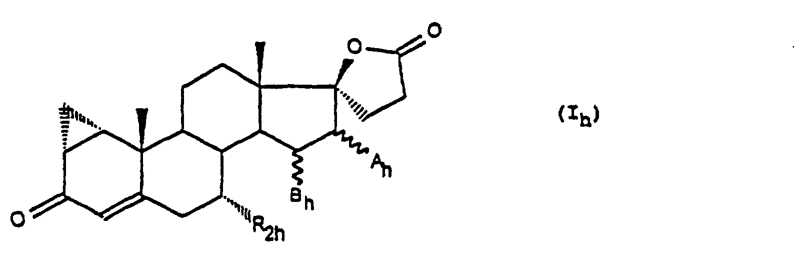 entsprechen, in der Aₕ, Bₕ und R₂ₕ jeweils die gleichen Werte besitzen wie A,B und R₂, wie sie in Anspruch 4 definiert sind.

18. Verwendung nach Anspruch 4, **dadurch gekennzeichnet, daß** die Verbindungen mit einer antimineralcorticoiden Aktivität der allgemeinen Formel (Iᵢ) 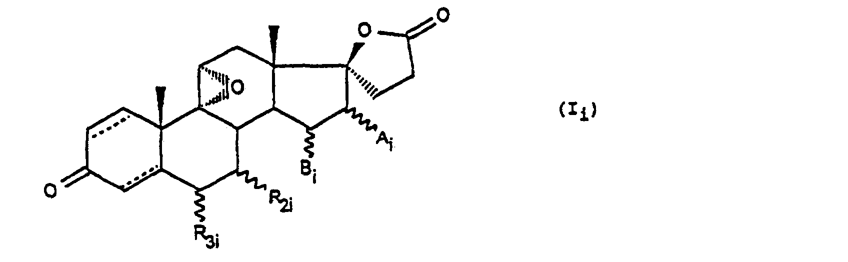 entsprechen, in der Aᵢ, Bᵢ, R₂ᵢ und R₃ᵢ jeweils die gleichen Werte besitzen wie A, B, R₂ und R₃, wie sie in Anspruch 4 definiert sind.

19. Verwendung nach Anspruch 4, **dadurch gekennzeichnet, daß** die Verbindungen mit einer antimineralcorticoiden Aktivität der allgemeinen Formel (Iⱼ) 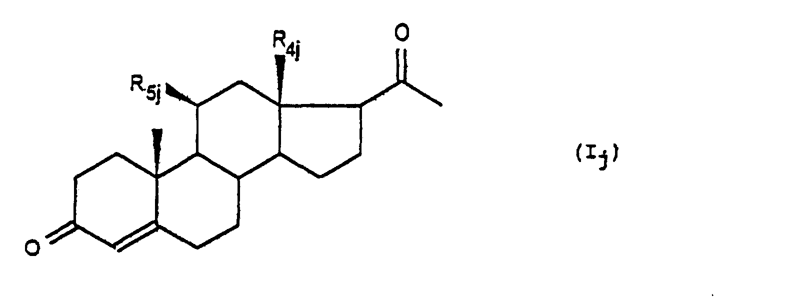 entsprechen, in der R₄ⱼ eine Gruppe Alkenyl oder Alkinyl mit 1 bis 8 Kohlenstoffatomen ist und R₅ⱼ einen Rest Hydroxyl oder ein Wasserstoffatom darstellt.

## Claims

1. Use of compounds having an antimineralocorticoid activity for the preparation of medicaments intended for the prevention or the treatment of the symptoms linked to drug dependency or spontaneous or precipitated drug withdrawal syndrome, caused by narcotics or a mixture of narcotics.

2. Use according to claim 1, **characterized in that** the narcotic is a morphinomimetic chosen from heroin, morphine and methadone.

3. Use according to claim 1, **characterized in that** the narcotic is cocaine.

4. Use according to any one of claims 1 to 3, **characterized in that** the compounds having an antimineralocorticoid activity correspond to general formula (I): 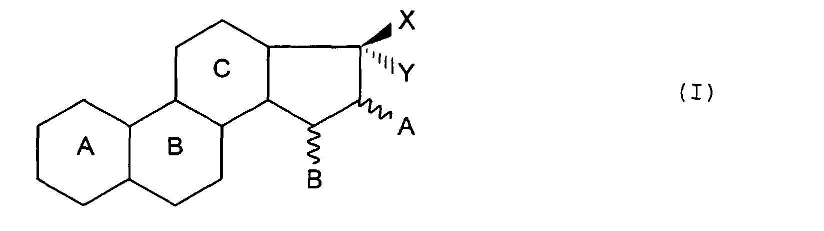 in which the rings A, B and C have one of the following structures: 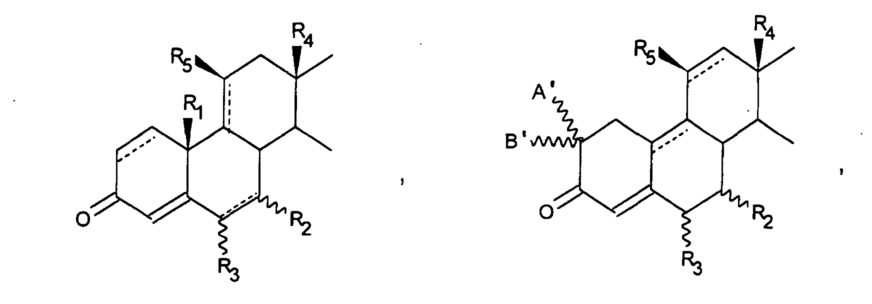 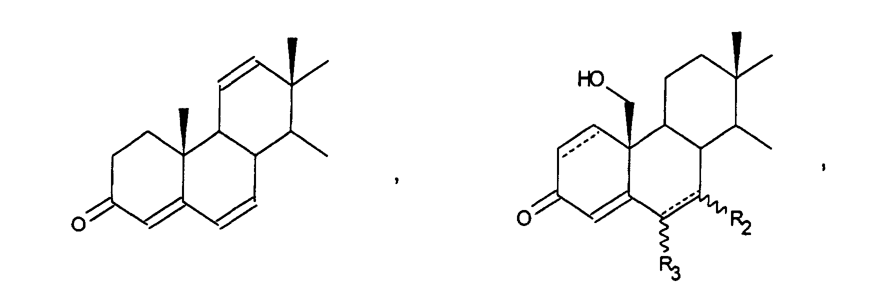 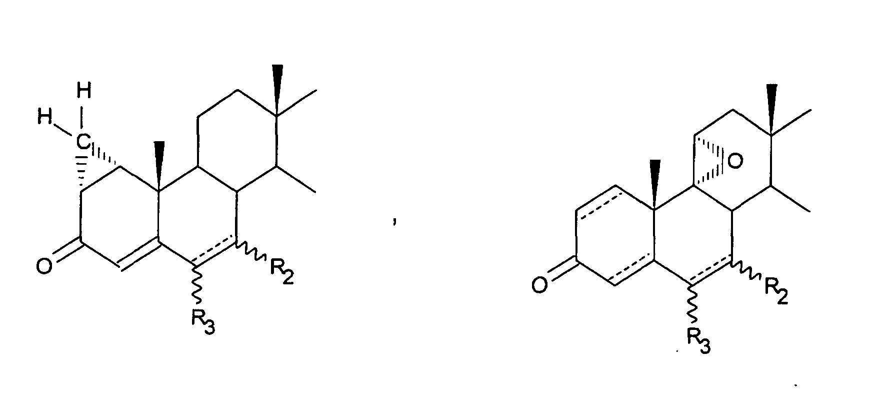 and in which:
either X and Y represent the: 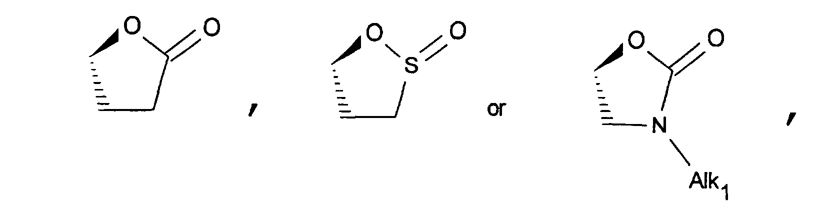 groups, Alk₁ representing an alkyl group containing at most 8 carbon atoms,
or X represents a hydroxyl, acetyloxy, propionyloxy, methoxy or ethoxy or ethoxy radical and Y represents a CH₂CH₂CO₂M, CH₂CH₂SO₂M or CH₂CH₂CH₂OH radical, M being a hydrogen atom, an alkali metal atom or an ammonium radical,
or X represents a COCH₂Z radical, in which Z represents a hydrogen atom, a hydroxyl radical or an acyloxy radical containing from 1 to 18 carbon atoms, and Y represents a hydrogen atom,
or X represents an 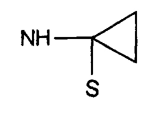 radical, S being an alkyl radical containing at most 8 carbon atoms, or a hydrogen atom and Y represents a hydrogen atom, A and B are hydrogen atoms or together form a methylene bridge in position 15α,16α or 15β,16β, A' and B' are hydrogen atoms, alkyl radicals containing from 1 to 4 carbon atoms, or form with the carbon which carries them a cyclopropyl radical, R₁ represents a methyl radical or a 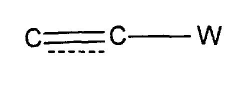 group, in which W represents
either a hydrogen atom,
or an alkyl radical containing at most 8 carbon atoms optionally substituted by a hydroxyl, free, esterified or salified carboxy, amino, tritylamino, chloroacetylamino, trifluoroacetylamino, halogen, monoalkylamino, dialkylamino radical, each alkyl radical containing at most 8 carbon atoms,
or an aryl or aralkyl radical containing at most 14 carbon atoms, optionally substituted by a hydroxyl, free, esterified or salified carboxy, amino, monoalkylamino, dialkylamino, alkyl, alkoxy or alkylthio radical, each alkyl radical containing at most 8 carbon atoms,
or a halogen atom,
or a trialkylsilyl radical, each alkyl radical containing at most 8 carbon atoms,
R₂ and R₃ are such that
either R₂ and R₃ together form a methylene bridge in position 6α,7α or 6β,7β,
or R₂ and R₃ are hydrogen atoms,
or R₃ is a hydrogen atom and R₂ represents a SCOCH₃, CO₂Alk group, Alk being an alkyl radical containing at most 8 carbon atoms, alkyl, alkenyl or alkynyl radical containing at most 8 carbon atoms and optionally substituted by a hydroxyl radical, free, esterified or salified carboxy, halogen, amino, monoalkylamino, dialkylamino radical, each alkyl radical containing at most 8 carbon atoms, R₄ represents an alkyl, alkenyl or alkynyl radical containing at most 8 carbon atoms, R₅ represents either an allenyl radical, or a hydroxyl radical, or a hydrogen atom, the dotted lines represent a second optional bond, the wavy lines indicate that the substituents are in position α or β, as well as the salts of the products of formula (I) with pharmaceutically acceptable acids and bases.

5. Use according to claim 4, **characterized in that** the compounds having an antimineralocorticoid activity correspond to general formula (Iₐ): 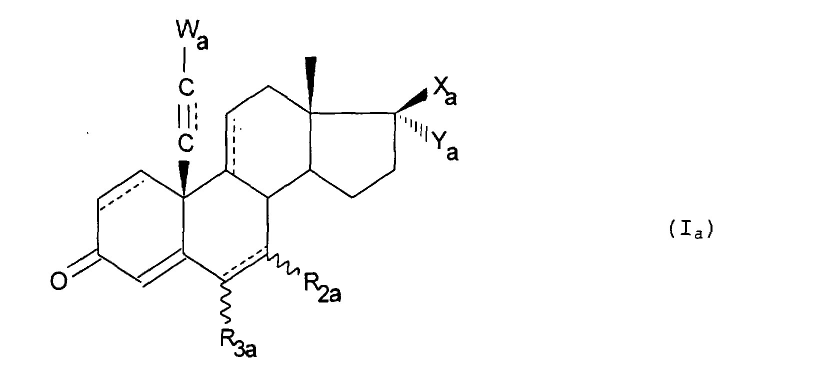 in which:
either Xₐ and Yₐ represent the 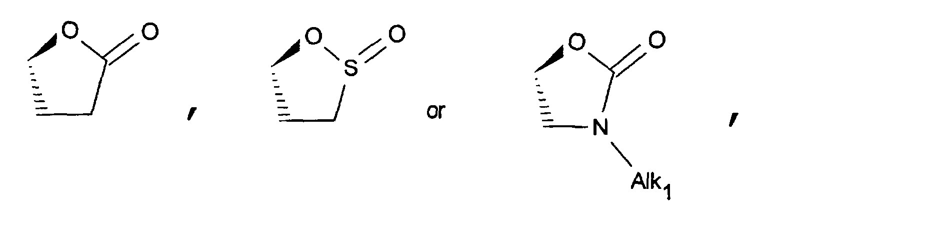 groups, Alk₁ being as defined in claim 4,
or Xₐ represents a hydroxyl, acetyloxy, propionyloxy, methoxy
or ethoxy radical and Yₐ represents a CH₂CH₂CO₂M, CH₂CH₂SO₂M or CH₂CH₂CH₂OH radical, M being as defined in claim 4,
R₂ₐ, R₃ₐ and Wₐ have the same values as R₂, R₃ and W
respectively as defined in 4 and the dotted or wavy lines have the meaning indicated in claim 4.

6. Use according to claims 4 or 5, **characterized in that** the compounds corresponding to general formulae (I) or (Iₐ) are chosen from the following list:
- γ-lactone of 10β-ethynyl,17β-hydroxy 3-oxo-19-nor-17α-pregna-4,9(11)-diene-21-carboxylic acid,
- γ-lactone of 17β-hydroxy 3-oxo-10β-(1-propynyl)-19-nor-17α-pregna-4,9(11)-diene-21-carboxylic acid,
- γ-lactone of 17β-hydroxy-3-oxo-10β-(1-propynyl)-19-nor-17α-pregn-4-ene-21-carboxylic acid,
- γ-lactone of 10β-ethynyl 17β-hydroxy 3-oxo-19-nor-17α-pregn-4-ene-21-carboxylic acid.

7. Use according to claim 4, **characterized in that** the compounds having an antimineralocorticoid activity correspond to general formula (I_{b}): 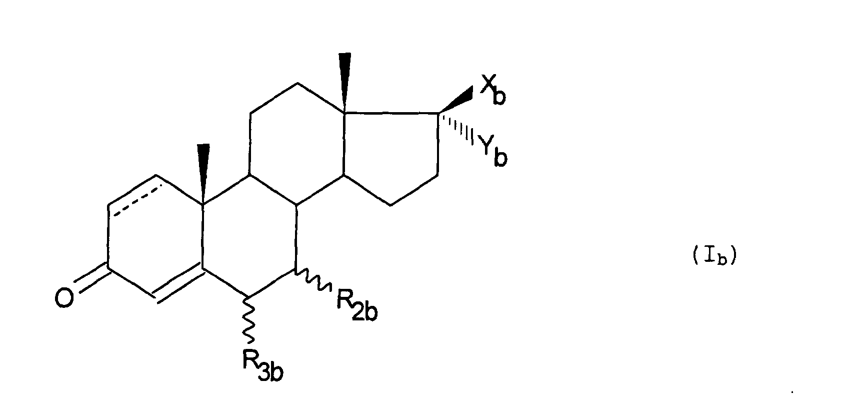 in which:
either X_{b} and Y_{b} represent the 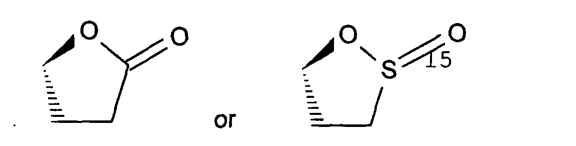 groups
or X_{b} represents a hydroxyl radical and Y_{b} represents a CH₂CH₂CO₂M radical, M being as defined in claim 4,
R_{2b} and R_{3b} are such that
either R_{2b} represents an optionally substituted alkyl, alkenyl or alkynyl radical as defined in claim 4 and R_{3b} is a hydrogen atom,
or R_{2b} and R_{3b} together form a methylene bridge in position 6α,7α or in position 6β,7β.

8. Use according to one of claims 4 or 7, **characterized in that** the compounds corresponding to general formulae (I) or (I_{b}) are chosen from the following list:
- (17R)-6β,7β-methylene-2'-oxydospiro-(androst-4-ene-17,5'-(1',2')-oxathiolane)-3-one
- (17R)-6α,7α-methylene-2'-oxydospiro-(androst-4-ene-17,5'-(1',2')-oxathiolane)-3-one
- (17R)-7α-methyl-2'-oxydospiro-(androst-4-ene-17,5'-(1',2')-oxathiolane)-3-one
- (17R)-7α-n-propyl-2'-oxydospiro-(androst-4-ene-17,5'-(1',2')-oxathiolane)-3-one
- γ-lactone of 17β-hydroxy-3-oxo-7α-propyl-17α-pregna-1,4-diene-21-carboxylic acid,
- potassium 17β-hydroxy-3-oxo-7α-propyl-17α-pregna-1,4-diene-21-carboxylate,
- γ-lactone of 17β-hydroxy-3-oxo-7α-propyl-(17α)-pregn-4-ene-21-carboxylic acid,
- potassium 17β-hydroxy-3-oxo-7α-propyl-(17α)-pregn-4-ene-21-carboxylate.

9. Use according to claim 4, **characterized in that** the compounds having an antimineralocorticoid activity, corresponding to general formula (I_{c}): 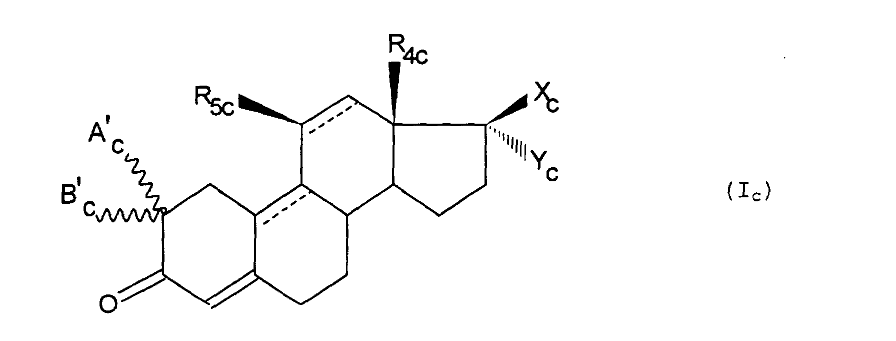 in which
either X_{c} and Y_{c} represent a 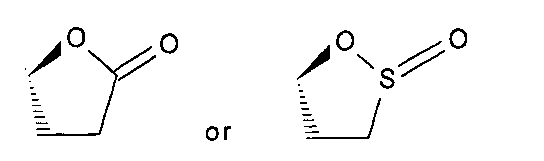 group
or X_{c} represents a hydroxyl radical and Y_{c} represents a CH₂CH₂CO₂M or CH₂CH₂SO₂M group, M being as defined in claim 4.
or X_{c} represents a COCH₂Z radical in which Z is as defined in claim 4 and Y_{c} is a hydrogen atom,
A'_{c} and B'_{c} have the same values as A' and B' respectively as defined in claim 4,
R_{4c} is a methyl or ethyl radical, R_{5c} is either a hydrogen atom, or an allenyl radical, it being understood that when R_{5c} is an allenyl radical A'_{c} and B'_{c} are hydrogen atoms, R_{4c} is a methyl radical, X_{c} and Y_{c} together form an 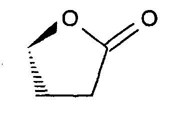 group, the dotted lines in position 9-10 form a second bond, and those in position 11-12 do not form a second bond.

10. Use according to one of claims 4 or 9, **characterized in that** the compounds corresponding to general formulae (I) or (I_{c}) are chosen from the following list:
- 2,2-dimethyl 19-nor pregn-4-ene-3,20-dione,
- 21-acetoxy 2,2-dimethyl 19-nor-pregn-4-ene 3,20-dione,
- 2,2-dimethyl 21-hydroxy 19-nor-pregn-4-ene 3,20-dione,
- 2,2-dimethyl 19-nor-pregna-4,9-diene 3,20-dione,
- 21-acetoxy 2,2-dimethyl 19-nor-pregna-4,9-diene 3,20-dione,
- 2,2-dimethyl 21-hydroxy 19-nor-pregna-4,9-diene 3,20-dione,
- 2,2-dimethyl 19-nor-pregna-4,9,11-triene 3,20-dione,
- 21-acetoxy 2,2-dimethyl 19-nor-pregna-4,9,11-triene 3,20-dione,
- 2,2-dimethyl 21-hydroxy 19-nor-pregna-4,9,11-triene 3,20-dione,
- (17R) 2'-oxydospiro-(estra-4,9-diene-17,5'-(1',2')-oxathiolane) 3-one,
- (17R) 2'-oxydospiro-(estra-4,9,11-triene-17,5'-(1',2')-oxathiolane) 3-one,
- (17R) 11β-hydroxy 2'-oxydospiro-(estra-4,9-diene-17,5'-(1',2')-oxathiolane) 3-one,
- 2,2-dimethyl-13-ethyl-21-hydroxy-18,19-dinor-pregn-4-ene 3,20-dione,
- γ-lactone of 11β-allenyl-17β-hydroxy-3-oxo-19-nor-17α-pregna-4,9-diene-21-carboxylic acid.

11. Use according to claim 4, **characterized in that** the compounds having an antimineralocorticoid activity correspond to general formula (I_{d}): 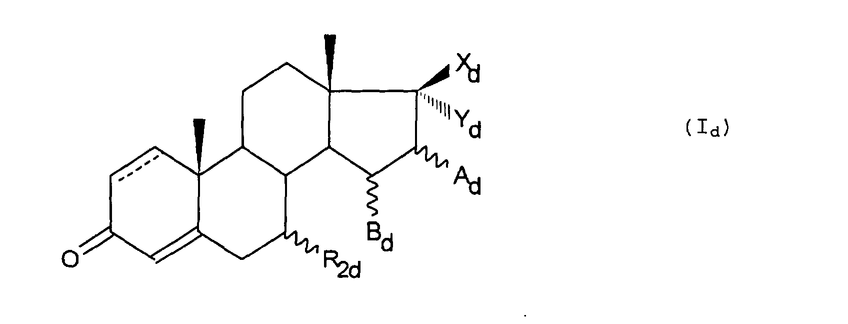 in which:
either X_{d} and Y_{d} represent the 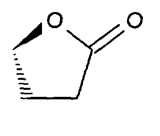 group,
or X_{d} represents a hydroxyl radical and Y_{d} represents a CH₂CH₂CO₂M radical, M being as defined in claim 4,
A_{d} and B_{d} have the same values as A and B respectively as defined in 4,
R_{2d} represents either a thioacetyl radical, or a CO₂Alk radical, Alk being an alkyl radical containing at most 8 carbon atoms and the dotted line in position 1-2 representing an optional double bond.

12. Use according to one of claims 4 or 11, **characterized in that** the compounds corresponding to general formula (I) or (I_{d}) are chosen from the following list:
- γ-lactone of 7α-acetylthio-17β-hydroxy-15β,16β-methylene-3-oxo-17α-pregna-1,4-diene-21-carboxylic acid,
- γ-lactone of 17β-hydroxy-7α-methoxycarbonyl-15β,16β-methylene-3-oxo-17α-pregn-4-ene-21-carboxylic acid,
- γ-lactone of 7α-acetylthio-17β-hydroxy-3-oxo-17α-pregn-4-ene-21-carboxylic acid,
- γ-lactone of 17β-hydroxy-7α-methoxycarbonyl-3-oxo-17α-pregn-4-ene-21-carboxylic acid,
- potassium salt of 17β-hydroxy-7α-methoxycarbonyl-3-oxo-pregn-4-ene-21-carboxylic acid.

13. Use according to claim 4, **characterized in that** the compounds having an antimineralocorticoid activity correspond to general formula (Iₑ): 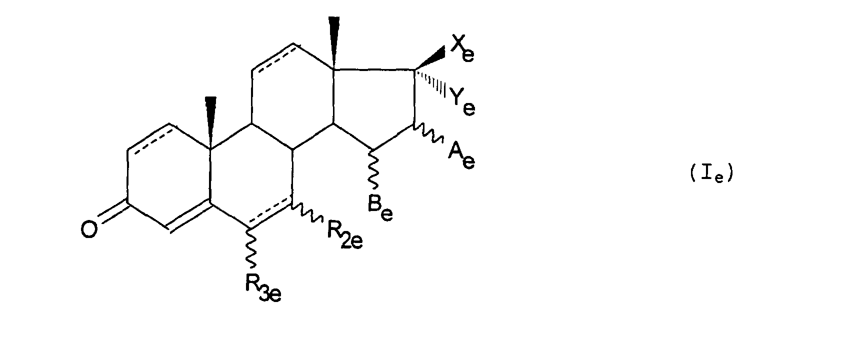 in which:
either Xₑ and Yₑ represent the 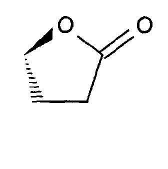 group,
or Xₑ represents a hydroxyl radical and Yₑ represents a CH₂CH₂CO₂M radical, M being as defined previously,
R₂ₑ or R₃ₑ are such that:
either R₂ₑ and R₃ₑ together form a methylene bridge in position 6α, 7α or 6β,7β,
or R₂ₑ and R₃ₑ are hydrogen atoms,
Aₑ and Bₑ have the same values as A and B respectively as defined in claim 4 and the dotted or wavy lines retain the same meaning as in claim 4.

14. Use according to one of claims 4 or 11, **characterized in that** the compounds corresponding to general formula (I) or (Iₑ) are chosen from the following list:
- γ-lactone of 17β-hydroxy-6β,7β,15β,16β-dimethylene-3-oxo-17α-pregna-1,4-diene-21-carboxylic acid,
- γ-lactone of 17β-hydroxy-6β,7β,15β,16β-dimethylene-3-oxo-17α-pregna-4-ene-21-carboxylic acid,
- γ-lactone of 17β-hydroxy-3-oxo-17α-pregna-4,6-diene-21-carboxylic acid,
- potassium salt of 17β-hydroxy-3-oxo-17α-pregna-4,6-diene-21-carboxylic acid,
- γ-lactone of 17β-hydroxy-3-oxo-17α-pregna-4,6,11-triene-21-carboxylic acid,
- γ-lactone of 17β-hydroxy-6β,7β-methylene-3-oxo-17α-pregn-4-ene-21-carboxylic acid,
- potassium salt of 17β-hydroxy-6β,7β-methylene-3-oxo-17α-pregn-4-ene-21-carboxylic acid.

15. Use according to claim 4, **characterized in that** the compounds having an antimineralocorticoid activity correspond to general formula (I_{f}): 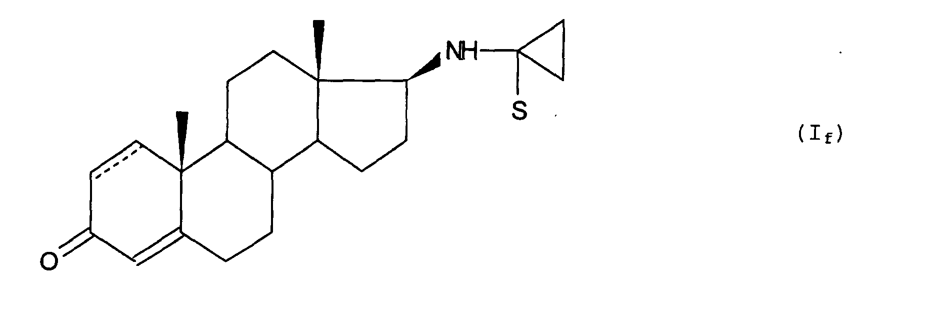 in which S represents an alkyl radical containing from 1 to 8 carbon atoms, or a hydrogen atom.

16. Use according to claim 4, **characterized in that** the compounds having an antimineralocorticoid activity correspond to general formula (I_{g}): 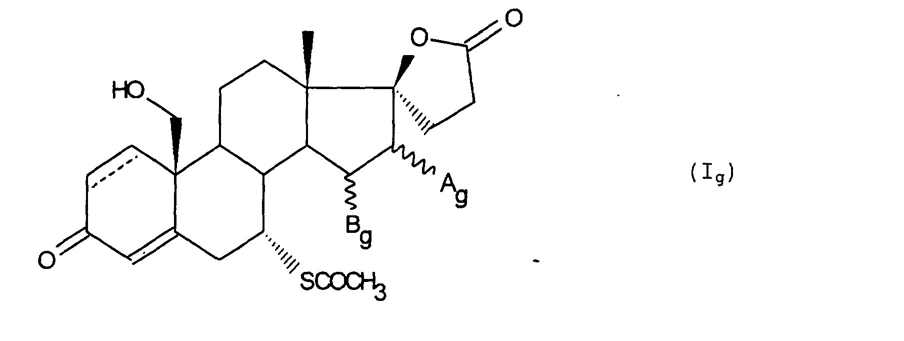 in which A_{g} and B_{g} have the same values as A and B respectively as defined in 4.

17. Use according to claim 4, **characterized in that** the compounds having an antimineralocorticoid activity correspond to general formula (Iₕ): 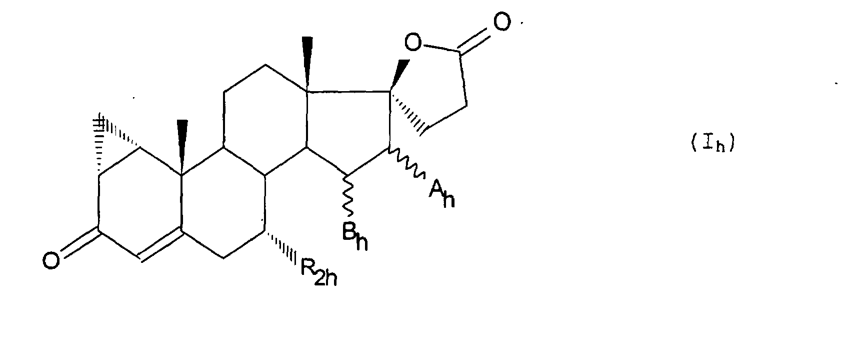 in which Aₕ, Bₕ and R₂ₕ have the same values as A, B and R₂ respectively as defined in claim 4.

18. Use according to claim 4, **characterized in that** the compounds having an antimineralocorticoid activity correspond to general formula (Iᵢ): 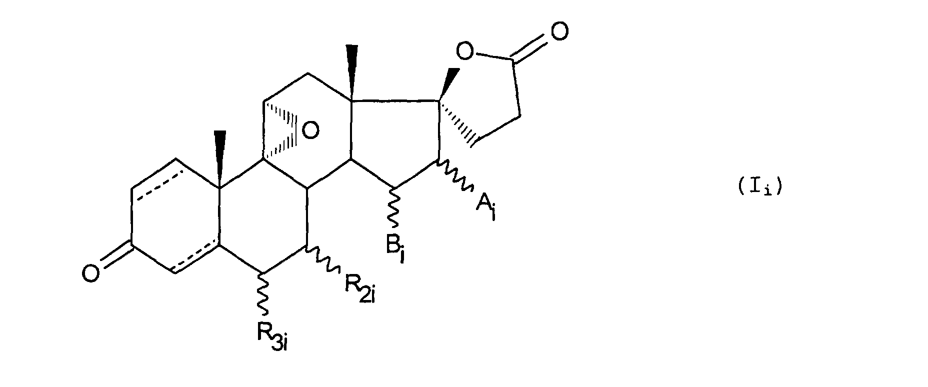 in which Aᵢ, Bᵢ, R₂ᵢ and R₃ᵢ have the same values as A, B, R₂ and R₃ respectively as defined in claim 4.

19. Use according to claim 4, **characterized in that** the compounds having an antimineralocorticoid activity correspond to general formula (Iⱼ): 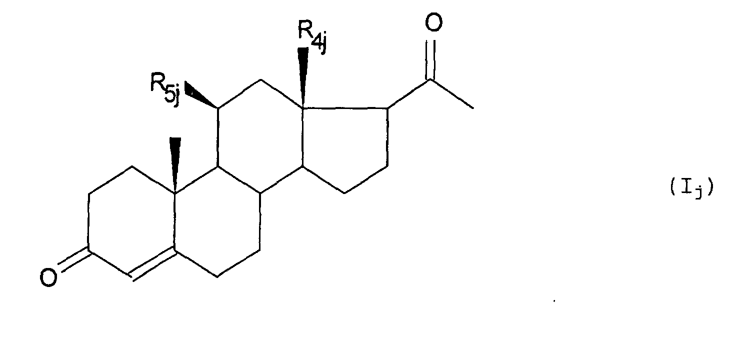 in which R₄ⱼ is a alkenyl or alkynyl group containing from 1 to 8 carbon atoms and R₅ⱼ is a hydroxyl radical or a hydrogen atom.
